# EUROPEAN PATENT APPLICATION

(11) **EP 3 056 512 A1**
(43) Date of publication of application: **17.08.2016**
(21) Application number: 15154521.7
(22) Date of filing: 10.02.2015
(51) Int. Cl.: C07K 16/28, C07K 16/46, A61K 39/395, A61K 39/00, C07K 16/16, C07K 16/18

(54) **Antibody construct**

(71) Applicant: Medizinische Universität Wien, 1090 Vienna (AT)
(72) Inventor: Madritsch, Christoph, 1180 Wien (AT); Flicker, Sabine, 1160 Wien (AT); Valenta, Rudolf, 2604 Theresienfeld (AT); Gadermaier, Elisabeth, 1170 Wien (AT); Eckl-Dorna, Julia, 1130 Wien (AT); Niederberger, Verena, 1080 Wien (AT); Blatt, Katharina, 2301 Groß-Enzersdorf (AT); Valent, Peter, 1180 Wien (AT); Ellinger, Isabella, 1160 Wien (AT)
(74) Representative: KLIMENT & HENHAPEL

(57) **Abstract**

The present invention relates to an antibody construct comprising at least one antigen binding site specific for ICAM1 and at least one antigen binding site specific for at least one allergen.

## Description

The present invention relates to an antibody construct comprising an antigen binding site specific for an allergen and uses thereof in the treatment and prevention of allergies.

IgE-associated allergies affect more than 25% of the population and hence represent a global health problem. Immediate allergic symptoms are caused by cross-linking of FcεRI on mast cells and basophils by IgE-allergen immune complexes. Current treatments to prevent or to reduce allergic symptoms, especially allergen-specific immunotherapy (SIT), aim to impede such a crosslinking and/or the binding of IgE molecules to allergens by induction of allergen-specific IgG antibodies. These treatments usually involve the administration of allergens, hypoallergenic variants of allergens or fragments of these polypeptides and proteins to patients. Since these molecules are usually administered by parenteral and thus invasive routes it is an object of the present invention to provide alternative means for the treatment or prevention of allergies or for the alleviation of allergic symptoms.

Therefore, the present invention relates to an antibody construct comprising at least one antigen binding site specific for ICAM1 and at least one antigen binding site specific for at least one allergen.

In order to reach immune cells which are resident in the target organs of allergic inflammation allergens must cross the epithelial barrier. Disturbances of the epithelial barrier function by endogenous factors (e.g. mutations in the filaggrin gene or chronic inflammation) and exogenous factors (e.g. damage of the epithelium by the proteolytic activity of allergens, rhinovirus infections and tobacco smoking) foster trans-epithelial allergen migration and subsequent allergic inflammation and may contribute to increases of local and systemic allergen-specific immune responses. Therefore, measures for improving barrier function, such as topical treatments of skin and nasal mucosa may reduce allergen uptake and allergic inflammation. It turned out that antibody constructs comprising binding sites specific for ICAM1 and an allergen are able to reduce significantly the allergen systemic uptake (i.e. transepithelial allergen migration) by capturing the allergen on the surface of epithelial cells, in particular respiratory epithelial cells. This prevents trans-epithelial allergen migration and, as a consequence, reduces allergic inflammation.

Intercellular adhesion molecule 1 (ICAM1; human ICAM1 has the sequence UniProt Acc. No. P05362), also known as CD54, is a type of intercellular adhesion molecule present in the membranes of, e.g., endothelial cells. ICAM1 present on the surface of cells is able to bind leukocytes enabling them to transmigrate into tissues. It was further characterized to be a site for the cellular entry of human rhinovirus. ICAM1 is markedly augmented by inflammatory mediators, including TNF-α, IL-1α and β, IFN-γ and endotoxins.

It has been reported that ICAM1 is highly expressed on the respiratory epithelium of allergic patients. Although ICAM1 is described to be responsible for the migration of molecules through tissues, it was surprisingly found that an antibody construct comprising an antigen binding site specific for ICAM1 and an antigen binding site specific for an allergen is useful for capturing allergens at the sites of tissue entry, thus preventing or significantly reducing the transmigration of allergens bound to said construct. The antibody construct of the present invention binds stably to the allergen as well as to ICAM1 on epithelial cells, in particular respiratory epithelial cells, and prevents trans-epithelial allergen migration. The complex comprising the antibody construct of the present invention and allergen formed on epithelial cells prevents the internalization of the allergen bound on the surface of these cells. Consequently, allergic inflammation can be significantly reduced.

The antibody construct of the present invention comprises at least one antigen binding site for ICAM1 and at least one antigen binding site for an allergen. In a preferred embodiment of the present invention the antibody construct of the present invention may comprise at least two, preferably at least three, more preferably at least five, antigen binding sites for ICAM1. In a further preferred embodiment of the present invention the antibody construct of the present invention may comprise at least two, preferably at least three, more preferably at least five, antigen binding sites for at least one, preferably at least two, more preferably at least five, allergen(s).

If the antibody construct of the present invention comprises more than one antigen binding sites for an allergen, the binding sites may be specific for one or more allergens. Thus, an antibody construct of the present invention may comprise antigen binding sites for one specific allergen or for more allergens. Such an antibody construct can be used to capture not only one allergen.

"Antibody construct" as used herein refers to any molecule, conjugate or complex comprising at least two different antigen binding sites enabling the construct to bind to, to interact with or to recognize the target molecules ICAM1 and an allergen.

"Conjugate" as used herein refers to at least a first antigen binding site or a first immunoglobulin or immunoglobulin fragment covalently bound to at least a second antigen binding site or a second immunoglobulin or immunoglobulin fragment, wherein the conjugate is able to bind to ICAM1 and an allergen. The parts of the conjugate of the present invention can be bound directly to each other or via a linker or spacer. A "linker" or "spacer" can be any chemical moiety that is capable of linking an antigen binding site, immunoglobulin or immunoglobulin fragment to at least another antigen binding site, immunoglobulin or immunoglobulin fragment in a stable, covalent manner. For cross-linking the antigen binding sites of the present invention methods known in the art can be used. For instance, these methods may involve the use of agents like MBS (m-Maleimidobenzoyl-N-hydroxysuccinimide ester), NHS(N-hydroxysuccinimide ester), EDC (1-ethyl-3-(3-dimethylaminopropyl) carbodiimide), glutaraldehyde, etc. These agents often require free functional groups (e.g. free SH-groups) in the molecules to be conjugated. In order to preserve the binding capacities of the antigen binding sites said binding sites should be ideally not affected by these agents.

"Complex" as used herein refers to at least two different antigen binding sites bound to each other in a non-covalent manner, e.g. by electrostatic, n-effects, van der Waals forces or hydrophobic effects.

The antibody construct of the present invention can also be a fusion protein. "Fusion protein" as used herein refers to a fusion of two or more amino acid sequences that are not naturally linked together. The fusion of the amino acid sequences can either be made by chemically linking ex vivo synthesized amino acid sequences. In an embodiment, the amino acid sequences are an in-frame translational fusion, i.e. correspond to a recombinant molecule expressed from a nucleic acid sequence in a prokaryotic or eukaryotic expression system. The term "fusion protein" as used herein refers also to a protein which may be created through genetic engineering from two or more proteins/peptides coding sequences joined together in a single polypeptide. In general, this is achieved by creating a "fusion gene", a nucleic acid that encodes and expresses the fusion protein. For example, a fusion gene that encodes a fusion protein may be made by removing the stop codon from a first DNA sequence encoding the first protein, then appending a DNA sequence encoding the second protein in frame. The resulting fusion gene sequence will then be expressed by a cell as a single fusion protein. Fusion proteins may include a linker (or "spacer") sequence which can promote appropriate folding and activity of each domain of the fusion protein. Fusion proteins may also include epitope tags for identification (e.g., in western blots, immunofluorescence, etc.) and/or purification. Non-limiting examples of epitope tags in current use include: HA-tag, myc-tag, FLAG-tag, HIS-tag (e.g. 6-HIS) and E-tag.

"Antigen binding site" or "binding site" as used herein denotes a region of an antigen binding molecule, preferably of a protein or a polypeptide, according to the invention to which a ligand (e.g. the antigen or antigen fragment of it) binds. The antigen binding site can have an artificial amino acid sequence obtainable by screening peptide libraries for peptides capable to bind to an antigen or may be derived from an antibody molecule or a fragment thereof. An antigen binding site can also be obtained from molecules like immunoglobulins. An antigen binding site is considered to specifically bind an antigen when the dissociation constant is preferably less than 1 µM, preferably less than 100 nM, more preferably less than 10 nM, even more preferably less than 1 nM, most preferably less than 100 pM.

As used herein, the term "specific for" can be used interchangeably with "binding to" or "binding specifically to". "Specific for" refers to molecules that bind to an antigen or a group of antigens with greater affinity (as determined by, e.g., ELISA or BIAcore assays) than other antigens or groups of antigens. According to the present invention molecules "specific for" an antigen may also be able to bind to more than one, preferably more than two, more preferably more than three, even more preferably more than five, antigens. Such molecules are defined to be "cross-reactive" (e.g. cross-reactive immunoglobulins, cross-reactive antigen binding sites). For example, molecules being "specific for" the birch pollen allergen Bet v 1 may also bind to the major allergens of alder, hazel etc.

The antibody construct of the present invention can be a complex, conjugate or fusion protein comprising an antigen binding site specific for ICAM1 and antigen binding site specific for an allergen. The antigen binding sites can be part of one or more immunoglobulins or fragments thereof. Thus, according to a preferred embodiment of the present invention the antibody construct is a complex, conjugate or fusion protein comprising an immunoglobulin or an immunoglobulin fragment specific for ICAM1 and an immunoglobulin or an immunoglobulin fragment specific for an allergen.

The antigen binding site of the antibody construct of the present invention may be part of or derived from an immunoglobulin (i.e. antibody). Therefore, the antibody construct of the present invention can be a complex, conjugate or fusion protein as defined herein comprising an immunoglobulin or an immunoglobulin fragment specific for ICAM1 and an immunoglobulin or an immunoglobulin fragment specific for an allergen. Exemplary immunoglobulins/antibodies or immunoglobulin/antibody fragments are selected from the group consisting of polyclonal antibodies, monoclonal antibodies, chimeric antibodies, humanized antibodies, human antibodies, multispecific antibodies, Fab, Fab', F(ab')2, Fv, domain antibody (dAb), complementarity determining region (CDR) fragments, CDR-grafted antibodies, single-chain antibodies (ScFv), single chain antibody fragments, chimeric antibodies, diabodies, triabodies, tetrabodies, minibodies, linear antibodies, chelating recombinant antibodies, tribodies, bibodies, intrabodies, nanobodies, small modular immunopharmaceuticals (SMIP), camelized antibodies, VHH containing antibodies and polypeptides that comprise at least a portion of an immunoglobulin that is sufficient to confer specific antigen binding to the polypeptide, such as one, two, three, four, five or six CDR sequences. In a particularly preferred embodiment of the present invention the immunoglobulin is a single-chain antibody.

It is particularly preferred that the antigen binding site or the immunoglobulin or fragments thereof comprising said site are recombinant molecules.

Methods of generating antibodies and antibody fragments are well known in the art. For example, antibodies may be generated via any one of several methods which employ induction of in vivo production of antibody molecules, screening of immunoglobulin libraries or generation of monoclonal antibody molecules by cell lines in culture. These include, but are not limited to, the hybridoma technique, the human B-cell hybridoma technique and the Epstein-Barr virus (EBV)-hybridoma technique.

Monoclonal antibodies specific for ICAM1 or an allergen may be prepared by known techniques, for example those disclosed in "Monoclonal Antibodies: A manual of techniques", H Zola (CRC Press, 1988). Antibody and immunoglobulin fragments can also be obtained using methods well known in the art (see, for example, Harlow and Lane, 1988, "Antibodies: A Laboratory Manual, Cold Spring Harbor Laboratory, New York). For instance, immunoglobulin fragments comprising the antigen binding sites of the present invention can be prepared by proteolytic hydrolysis of antibodies or by recombinant expression in eukaryotes or prokaryotes such as E. coli, mammalian (e.g. CHO cells), insect cells or yeast cells (e.g. P. pastoris) of DNA encoding the fragment comprising said antigen binding sites. Alternatively, antibody fragments can be obtained by pepsin or papain digestion of whole antibodies by conventional methods.

Preferably, the antibody or antigen-binding fragment thereof is a human antibody or humanized antibody. For human therapy humanized antibodies may be used. Humanized forms of non-human (e.g. murine) antibodies are genetically engineered chimeric antibodies or antibody fragments having minimal-portions derived from non-human antibodies. Humanized antibodies include antibodies in which complementarity determining regions of a human antibody (recipient antibody) are replaced by residues from a complementarity determining region of a non-human species (donor antibody) such as mouse, rat or rabbit having the desired functionality. In some instances, Fv framework residues of the human antibody are replaced by corresponding non-human residues. Humanized antibodies may also comprise residues which are found neither in the recipient antibody nor in the imported complementarity determining region or framework sequences. In general, humanized antibody will comprise substantially all or at least one, and typically two, variable domains, in which all or substantially all of the complementarity determining regions correspond to those of a non-human antibody and all, or substantially all, of the framework regions correspond to those of a relevant human consensus sequence. Humanized antibodies may also include at least a portion of an antibody constant region, such as an Fc region, typically derived from a human antibody. Methods for humanizing non-human antibodies are well known in the art. Generally, the humanized antibody has one or more amino acid residues introduced into it from a source which is non-human. Humanization can be performed, for instance, by substituting human complementarity determining regions with corresponding rodent complementarity determining regions. Accordingly, such humanized antibodies are chimeric antibodies, wherein substantially less than an intact human variable domain has been substituted by the corresponding sequence from a non-human species. In practice, humanized antibodies may be typically human antibodies in which some complementarity determining region residues and possibly some framework residues are substituted by residues from analogous sites in rodent antibodies.

Regardless of structure, an "immunoglobulin fragment" of the present invention binds with the same antigen that is recognized by the full-length immunoglobulin. For instance, antibody fragments may include isolated fragments consisting of the variable regions, such as the "Fv" fragments consisting of the variable regions of the heavy and light chains or recombinant single chain polypeptide molecules in which light and heavy variable regions are connected by a peptide linker ("ScFv proteins"). The term "fragment" refers to a portion of a polypeptide typically having at least 10 contiguous or at least 20 contiguous or at least 50 contiguous amino acids of the immunoglobulin. The "immunoglobulin fragment" has to consist at least of or to comprise at least an antigen binding site. Exemplary antigen binding fragments may be selected from the group consisting of Fv fragments (e.g. single chain Fv and disulphide-bonded Fv), and Fab-like fragments (e.g. Fab fragments, Fab' fragments and F(ab)₂ fragments).

In one preferred embodiment of the present invention the antigen binding fragment is an ScFv. One advantage of using antibody fragments, rather than whole antibodies, is the simple and efficient recombinant expression of antigen binding fragments such as Fab, Fv, ScFv and dAb antibody fragments.

According to a preferred embodiment of the present invention the antibody construct of the present invention comprises an antigen binding site, an immunoglobulin or an immunoglobulin fragment specific for ICAM1 chemically cross-linked to an antigen binding site, an immunoglobulin or an immunoglobulin fragment specific for an allergen.

According to a particularly preferred embodiment of the present invention the antibody construct is a bispecific antibody, a bispecific diabody or a bispecific (Fab)₂ fragment, whereby a bispecific diabody comprising two single chain variable fragments (ScFv) is most preferred.

Bispecific antibodies, diabodies and (Fab)₂ fragments can be obtained by methods known in the art (see e.g. Chames and Baty Curr Opin Drug Disc Dev 12(2009):276).

Exemplary bispecific molecules of the invention comprise a single antibody that has two arms comprising different antigen binding sites, a single antibody that has one antigen-binding region or arm specific to ICAM1 and a second chain or arm specific to an allergen, a single chain antibody that has specificity to ICAM1 and an allergen, e.g., via two ScFvs linked in tandem by a (peptide) linker; a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In: Antibody Engineering, Springer Berlin Heidelberg (2010)), a chemically-linked bispecific (Fab')2 fragment, a Tandab, which is a fusion of two single chain diabodies resulting in a tetravalent bispecific antibody that has two binding sites for each of the target antigens, a flexibody, which is a combination of ScFvs with a diabody resulting in a multivalent molecule , a so called "dock and lock" molecule, based on the "dimerization and docking domain" in Protein Kinase A, which, when applied to Fabs, can yield a trivalent bispecific binding protein consisting of two identical Fab fragments linked to a different Fab fragment, a so-called Scorpion molecule, comprising, e.g., two ScFvs fused to both termini of a human Fab-arm, and a diabody.

Methods for preparing bispecific antibodies are known to the person skilled in the art (see e.g. WO 2008/119353, WO 2011/131746). Examples of other platforms useful for preparing bispecific antibodies include but are not limited to BITE (Micromet), DART (MacroGenics), Fcab and Mab2 (F-star) and Fcengineered IgG₁ (Xencor). Of course, methods involving hybrid hybridoma and chemical conjugation (Marvin and Zhu Acta Pharmacol Sin 26(2005):649) can also be used. Co-expression in a host cell of two antibodies, consisting of different heavy and light chains, leads to a mixture of possible antibody products in addition to the desired bispecific antibody, which can then be isolated by, e.g., affinity chromatography or similar methods.

The antibody construct comprising antigen binding sites specific for ICAM1 and at least one allergen preferably comprises or consists of an intact antibody. Alternatively, the antibody construct comprises or consists of portions of intact antibodies sufficient to display binding specificity for ICAM1 and at least one allergen.

In an alternative embodiment, the antibody construct comprises or consists of antigen binding fragment selected from the group consisting of a Fv fragment, an Fab fragment, an Fab-like fragment and combinations thereof.

It is known in the art that ICAM1 is highly expressed on the respiratory epithelium of allergic patients and in the oral and gut epithelium. Therefore, it is particularly preferred to provide antibody constructs which comprise next to one or more antigen binding sites specific for ICAM1 one or more antigen binding sites specific for allergens which are characterized to be inhalation or food allergens.

"Inhalation allergens" are allergens which occur in nature in a form so that these allergens can be inhaled by a mammal or a human (e.g. pollen). Thus, "inhalation allergens" get usually in contact with respiratory epithelial cells present, for instance, in the lung and in tracheae and/or also with the surface of the eye.

"Food allergens", as used herein, refers to allergens which are present in food ingested by a mammal or a human. "Food allergens", thus, get in contact with the mouth (oral epithelium), with the esophagus and the gut (gut epithelilum).

According to a particularly preferred embodiment of the present invention the inhalation allergen is selected from the group consisting of pollen allergens, mold allergens, mite allergens and animal allergens, preferably pet allergens.

According to a further embodiment of the present invention the allergen is selected from the group consisting of a pollen allergen, mite allergen, cat allergen, dog allergen, insect bite/sting allergen, legume allergen, fruit allergen, nut allergen, milk allergen and fish allergen.

The allergen to which the antigen binding site of the antibody construct of the present invention binds to or is specific for is preferably a pollen allergen selected from the group of a grass pollen allergen, preferably the grass pollen allergen Phl p 1, Phl p 2, Phl p 4, Phl p 5, Phl p 6, Phl p 7, Phl p 11, Phl p 12 or Phl p 13, a weed allergen, preferably the ragweed allergen Amb a 1, Amb a 2, Amb a 3, Amb a 4, Amb a 5, Amb a 6, Amb a 7, Amb a 8, Amb a 9 or Amb a 10, a mugwort allergen, preferably Art v 1, Art v 2, Art v 3, Art v 4, Art v 5 or Art v 6, a Paritaria pollen allergen, preferably Par j 1, Par j 2, Par j 3 or Par j 4, an olive pollen allergen, preferably Ole e 1, Ole e 2, Ole e 3, Ole e 4, Ole e 5, Ole e 6, Ole e 7, Ole e 8, Ole e 9, Ole e 10 or Ole e 11, a pollen allergen from Saltwort thistle, preferably Sal k 1, Sal k 2, Sal k 3, Sal k 4 or Sal k 5, and a tree pollen, preferably a Cypress pollen, preferably Cha o 1 or Cha o 2, or a cedar pollen, preferably Jun a 1, Jun a 2 or Jun a 3, or a birch pollen allergen, preferably Bet v 1, whereby Phl p 1, Phl p 2, Phl p 5 and Bet v 1 are most preferred.

According to a particularly preferred embodiment of the present invention the allergen is Phl p 2 so that the antigen binding site of the antibody construct is specific for and binds to Phl p 2.

According to a preferred embodiment of the present invention the antibody construct comprises an antigen binding site or immunoglobulin or immunoglobulin fragment specific for Phl p 2, comprising an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 and combinations thereof.

SEQ ID NO: 1:
LESGPGLVKPAQTLSLSCAVSGGSIRSGGYYWSWIRQHPGKGLEWIGYIYHSGN
TYYNPSLKSRIAMSVDTSENKFSLRLNSVTAADTAVYYCARLDGYTLDIWGQGT
LVTVSS

SEQ ID NO: 2:
ELTQSPSSLSASVGDRVTISCRASQRINTYLNWYQHKPGKAPKLLIYAASSLQS
GVPSRFSGSGYGTDFTLTISSLQPEDFASYYCQESLSASYTFGQGTKVEIKR

SEQ ID NO: 3:

MAQVQLQESGPGLVKPAQTLSLSCAVSGGSIRSGGYYWSWIRQHPGKGLEWIGY
IYHSGNTYYNPSLKSRIAMSVDTSENKFSLRLNSVTAADTAVYYCARLDGYTLD
IWGQGTLVTVSSGGGGSGGGGSGGGGSETTLTQSPSSLSASVGDRVTISCRASQ
RINTYLNWYQHKPGKAPKLLIYAASSLQSGVPSRFSGSGYGTDFTLTISSLQPE
DFASYYCQESLSASYTFGQGTKVEIKRAAAGAPVPYPDPLEPR

SEQ ID NO: 4:

LESGPGLVKPSQTLSLTCTVSGGSIRSGGYYWSWVRQPPGKGLEWIGNIYHSGN
TYYNPSLKSRITMSVDTSKNHFSLRLTSVTAADTAVYYCARSDGYTLDNWGQGT
LVTVSS

SEQ ID NO: 5:
ELTQSPSSLSASVGDRVTITCRARQSISTYLNWYQQKPGKAPKLLIWSASNLQS
GVPSRFSGSGSGTEFTLTISNLQPEDFASYYCQQSYTTLYTFGPGTKLEIKR

SEQ ID NO: 6:
LESGPGLVKPSQTLSLTCTVSGGSIRSGGYYWSWIRQPPGKGLEWIGYIYHSGN
TYYNPSLKSRVTMSVDTSKNHFSLRLSSVTAADTAVYYCARSDGYTLDNWGQGT
LVTVSS

SEQ ID NO: 7:
ELTQSPSSVSASVGDRVTITCRASQGISSWLAWYQQKPGKAPKLLIYSASSLQS
GVPSRFSGSGSGTDFSLTISSLQPEDSATYYCQQANSFPYTFGQGTKVEIKR

The antibody construct of the present invention able to bind to Phl p 2 comprises preferably SEQ ID NO: 1 and 2, SEQ ID NO: 3, SEQ ID NO: 4 and 5, SEQ ID NO: 6 and 7, or combinations thereof.

According to a preferred embodiment of the present invention the immunoglobulin specific for Phl p 2 comprises at least one CDR sequence selected from the group consisting of SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19. The immunoglobulin specific for Phl p 2 comprises preferably CDR sequences SEQ ID NO: 8, SEQ ID NO: 9 and SEQ ID NO: 10; and/or SEQ ID NO: 11, SEQ ID NO: 12 and SEQ ID NO: 13; and/or SEQ ID NO: 8, SEQ ID NO: 9 and SEQ ID NO: 14; and/or SEQ ID NO: 15, SEQ ID NO: 16 and SEQ ID NO: 17; and/or SEQ ID NO: 18, SEQ ID NO: 16 and SEQ ID NO: 19. The immunoglobulin specific for Phl p 2 comprises preferably a heavy chain comprising CDR sequences SEQ ID NO: 8, SEQ ID NO: 9 and SEQ ID NO: 10; and a light chain comprising CDR sequences SEQ ID NO: 11, SEQ ID NO: 12 and SEQ ID NO: 13; a heavy chain comprising CDR sequences SEQ ID NO: 8, SEQ ID NO: 9 and SEQ ID NO: 14; and a light chain comprising CDR sequences SEQ ID NO: 15, SEQ ID NO: 16 and SEQ ID NO: 17; or a heavy chain comprising CDR sequences SEQ ID NO: 8, SEQ ID NO: 9 and SEQ ID NO: 14; and a light chain comprising CDR sequences SEQ ID NO: 18, SEQ ID NO: 16 and SEQ ID NO: 19 (see Table A below).

According to a further preferred embodiment of the present invention the antibody construct comprises an antigen binding site or immunoglobulin or immunoglobulin fragment specific for Phl p 1, comprising an amino acid sequence selected from the group consisting of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and combinations thereof.

SEQ ID NO: 20:
LESGGGWVQPGRSLRLSCAASGFTFDDHAMHWVRQAPGKGLEWVAGVSWNSGTR
AYADSVKGRFTISRDNAKNSLYLEMKSLRAEDTAIYYCARDMRAATPYSFDYWG
QGVLVTVSS

SEQ ID NO: 21:
ELVMTQSPSSLSASVGDRVTITCRASQTFNNYLNWYQQKPGKAPNLLIYAASTL
RRGIPSRFSGSGSGTYFTLTINGLQPEDFATYYCQQSYSTPLTFGGGTKVDIKR

SEQ ID NO: 22:
LESGGGLVQPGGSLRLSCAASGFTFDDHAMHWVRQTPEKGLQWVSGISWNSGVV
AYADSVKGRFSISRDNAKNSLYLQMNNLIPEDTAVYYCVRDFRAASPYFFDYWG
QGTRVTVSS

SEQ ID NO: 23:
ELTQSPSSLSASVGDRVTITCRASQSIGNYLNWYQQKPGKAPNLLIYAASSLQS
GVPSRFSGSGSGTDFTLTISSLQREDFATYYCQQSNRTPITFGQGTRLEIKG

SEQ ID NO: 24:
MAQVNLRESGGGLVQPGGSLRLSCAASGFTFDDHAMHWVRQTPEKGLQWVSGIS
WNSGVVAYADSVKGRFSISRDNAKNSLYLQMNNLIPEDTAVYYCVRDFRAATPY
FFDYWGQGTLVTVSSGGGGSGGGGSGGGGSETTLTQSPSSLSASVGDRVTITCR

ASQSIGNYLNWYQQKPGKAPNLLIYAASSLQSGVPSRFSGSGSGTDFTLTISSL
QREDFATYYCQQSNRTPITFGQGTRLEIKRAAAGAPVPYPDPLEPR

SEQ ID NO: 25:
LESGGGWVQPGRSLRLSCSASGLTFDDYAMHWVRQAPGKGLEWVSGISWNSGVR
AYADSVKGRFTISRDNGKNSLYLQMNSLRPEDTALYYCAKDIRAATPYALDHWG
QGVLVTVSS

SEQ ID NO: 26:
ELVMTQSPSSLSASEGDRVTITCRASRTIYNYLNWYQQKPGRAPKLLIHAASTL
QDGVPSRFSGSGSGTEFTLTISGLQSEDFATYYCQQSHGTPLTFGGGTTVDVKR

SEQ ID NO: 27:

MAQVQLQESGGGLVQPGGSLKLSCAASEFTFNNYWMSWVRQAPGKGLEWVANIK
PDGSVKYYVDSVKGRFTISRDNAKNSLFLQMNSLRAEDTAVYFCARERQSGAFG
LWGQGTTVTVSSGAGGSGGGGSGGGGSDIQMTQSPSSLSASVGDRVTITCRASQ
SISSYLNWYQQKPGKAPKLLIYAASSLQSGVPSRFSGSGSGTDFTLTISSLQPE
DFATYYCQQSYSTPLTFGGGTKVEIKRAAAGAPVPYPDPLEPR

SEQ ID NO: 28:

MAQVNLRESGGGLVRPGRSLRLSCAASGFTFDDYAMHWVRQAPGKGLEWVSGIS
WNSGSIGYADSVKGRFTISRDNAKNSLYLQMNSLRAEDTAVYYCAREWDGTAYY
GYTVGYWGQGTLVTVSSGGGGSGGGGSGGGGSDVVMTQSPLSLPVTLGQPASIS
CRSSQSLVYSDGNTYLNWFQQRPGQSPRRLIYKVSNRDSGVPDRFSGSGSGTDF
TLKISRVEAEDVGVYYCMQGTHWPPTFGQGTRLEIKRAAAGAPVPYPDPLEPR

SEQ ID NO: 29:

MAQVQLQESGGGVVQPGRSLRLSCAASGFTFSSYAMSWVRQAPGKGLEWVSGIS
GSGGSTYYADSVKGRFTISRDNSKNTLYLQMNSLRAEDTAIYYCARVLRNQAAA
GTIRWFDPWGQGTLVTVSSGGGGSGGGGSGGGGSDVVMTQSPLSLPVTLGQPAS
ISCRSSQSLVYSDGNTYLNWFQQRPGQSPRRLIYKVSNRDSGVPDRFSGSGSGT
DFTLKIRRVEAEDVGVYYCMQGTHWPPTFGQGTKLEIKRAAAGAPVPYPDPLEP
R

The antibody construct of the present invention able to bind to Phl p 1 comprises preferably SEQ ID NO: 20 and 21, SEQ ID NO: 22 and 23, SEQ ID NO: 24, SEQ ID NO: 25 and 26, SEQ ID NO: 27, SEQ ID NO: 28, SEQ ID NO: 29 or combinations thereof.

According to a preferred embodiment of the present invention the immunoglobulin specific for Phl p 1 comprises at least one CDR sequence selected from the group consisting of SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54 , SEQ ID NO: 55, SEQ ID NO: 56 and SEQ ID NO: 57. The immunoglobulin specific for Phl p 1 comprises preferably CDR sequences SEQ ID NO: 30, SEQ ID NO: 31 and SEQ ID NO: 32; and/or SEQ ID NO: 33, SEQ ID NO: 12 and SEQ ID NO: 34; and/or SEQ ID NO: 30, SEQ ID NO: 35 and SEQ ID NO: 36; and/or SEQ ID NO: 37, SEQ ID NO: 12 and SEQ ID NO: 38; and/or SEQ ID NO: 39, SEQ ID NO: 40 and SEQ ID NO: 41; and/or SEQ ID NO: 42, SEQ ID NO: 12 and SEQ ID NO: 43; and/or SEQ ID NO: 44, SEQ ID NO: 45 and SEQ ID NO: 46; and/or SEQ ID NO: 47, SEQ ID NO: 12 and SEQ ID NO: 34; and/or SEQ ID NO: 48, SEQ ID NO: 49 and SEQ ID NO: 50; and/or SEQ ID NO: 51, SEQ ID NO: 52 and SEQ ID NO: 53; and/or SEQ ID NO: 54, SEQ ID NO: 55 and SEQ ID NO: 56; and/or SEQ ID NO: 57, SEQ ID NO: 52 and SEQ ID NO: 53. The immunoglobulin specific for Phl p 1 comprises preferably a heavy chain comprising CDR sequences SEQ ID NO: 30, SEQ ID NO: 31 and SEQ ID NO: 32; and a light chain comprising CDR sequences SEQ ID NO: 33, SEQ ID NO: 12 and SEQ ID NO: 34; a heavy chain comprising CDR sequences SEQ ID NO: 30, SEQ ID NO: 35 and SEQ ID NO: 36; and a light chain comprising CDR sequences SEQ ID NO: 37, SEQ ID NO: 12 and SEQ ID NO: 38; or a heavy chain comprising CDR sequences SEQ ID NO: 39, SEQ ID NO: 40 and SEQ ID NO: 41; and a light chain comprising CDR sequences SEQ ID NO: 42, SEQ ID NO: 12 and SEQ ID NO: 43; or a heavy chain comprising CDR sequences SEQ ID NO: 44, SEQ ID NO: 45 and SEQ ID NO: 46; and a light chain comprising CDR sequences SEQ ID NO: 47, SEQ ID NO: 12 and SEQ ID NO: 34; or a heavy chain comprising CDR sequences SEQ ID NO: 48, SEQ ID NO: 49 and SEQ ID NO: 50; and a light chain comprising CDR sequences SEQ ID NO: 51, SEQ ID NO: 52 and SEQ ID NO: 53; or a heavy chain comprising CDR sequences SEQ ID NO: 54, SEQ ID NO: 55 and SEQ ID NO: 56; and a light chain comprising CDR sequences SEQ ID NO: 57, SEQ ID NO: 52 and SEQ ID NO: 53 (see Table A below).

According to a further preferred embodiment of the present invention the antibody construct comprises an antigen binding site or immunoglobulin or immunoglobulin fragment specific for Phl p 5, comprising an amino acid sequence selected from the group consisting of SEQ ID NO: 72, SEQ ID NO: 73, SEQ ID NO: 74, SEQ ID NO: 75, SEQ ID NO: 76 and combinations thereof.

SEQ ID NO: 72:

LESGGGVVQPGRSLRLSCAASEFAFDSYEIYWVRQAPGKGLEWVAMISIDETNK
HYADSVKGRFTIFRDNSNKTVHLQMNNLRSEDSGVYYCARWNNWNHRGMVFAFD
LWGQGTMVTVSS

SEQ ID NO: 73:

ELTQSPSSVSASVGDRVTITCRASQGISSWLAWYQQKPGKAPKLLIYSASSLQS
GVPSRFSGSGSGTDFSLTISSLQPEDSATYYCQQANSFPYTFGQGTKVEIKR

SEQ ID NO: 74:

ELVMTQSPSSVSASVGDRVTITCRASHSISNYLNWYQQKPGKAPKLLIYAASSL
QSGVPSRFSGSGSGTDFTLTISSLQPEDFANYYCQESFSPSGTFGQGTKVEIKR

SEQ ID NO: 75:

ELTQSPSSLSASVGDRVTITCRASQSILGYLNWYQQKPGKAPKLLIYAASTLQS
GVPSRFSGSGSGTDFTLTITSLQPDDFATYYCQQSYITPRTFGQGTKVEVKR

SEQ ID NO: 76:

ELVMTQSPSSLSAFVGDRVTITCRASQSISSYLNWYQQKPGKAPKLLIYAASSL
QSGVPSRFSGSGSGTDFTLTISSLQPEDFATYYCQQSHSTPYTFGQGTNLEIKR

The antibody construct of the present invention able to bind to Phl p 5 comprises preferably SEQ ID NO: 72 and 73, SEQ ID NO: 74, SEQ ID NO: 75, SEQ ID NO: 76 or combinations thereof.

According to a preferred embodiment of the present invention the immunoglobulin specific for Phl p 5 comprises at least one CDR sequence selected from the group consisting of SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 12, SEQ ID NO: 16, SEQ ID NO: 19 and SEQ ID NO: 47. The immunoglobulin specific for Phl p 5 comprises preferably CDR sequences SEQ ID NO: 77, SEQ ID NO: 78 and SEQ ID NO: 79; SEQ ID NO: 80, SEQ ID NO: 16 and SEQ ID NO: 19; and/or SEQ ID NO: 81, SEQ ID NO: 12 and SEQ ID NO: 82; and/or SEQ ID NO: 83, SEQ ID NO: 12 and SEQ ID NO: 84; and/or SEQ ID NO: 47, SEQ ID NO: 12 and SEQ ID NO: 85. The immunoglobulin specific for Phl p 5 comprises preferably a heavy chain comprising CDR sequences SEQ ID NO: 77, SEQ ID NO: 78 and SEQ ID NO: 79; and a light chain comprising CDR sequences SEQ ID NO: 80, SEQ ID NO: 16 and SEQ ID NO: 19; or a light chain comprising CDR sequences SEQ ID NO: 81, SEQ ID NO: 12 and SEQ ID NO: 82; or a light chain comprising CDR sequences SEQ ID NO: 83, SEQ ID NO: 12 and SEQ ID NO: 84; or a light chain comprising CDR sequences SEQ ID NO: 47, SEQ ID NO: 12 and SEQ ID NO: 85.

According to a further preferred embodiment of the present invention the antibody construct comprises an antigen binding site or immunoglobulin or immunoglobulin fragment specific for Phl p 6, comprising an amino acid sequence selected from the group consisting of SEQ ID NO: 86, SEQ ID NO: 87, SEQ ID NO: 88 and combinations thereof.

SEQ ID NO: 86:

EVQLVQSGGGLVKPGGSLRLSCAASGFIFSDHYMAWVRQTPGKGLEWISYISMT
SSYTNYADSVKGRFTISRDNAKNSLSLEMNSLRVEDTAVYYCARDRLRNWNDDA
FDIWGPGTMVTVSSGGGGSGGGGSGGGGSSYELTQPASVSGSPGQSITISCTGT
PSDIGTYPYVSWYQQHPDRAPKLLIYGVTNRPSGVSNRFSGSKSGNTASLTISE
LQAEDEADYYCSSYTLTSIVVFGGGTKLTVLGDYKDDDDK

SEQ ID NO: 87:

EVQLVQSGGGLVKPGGSLRLSCAASGFIFSDHYMAWVRQTPGKGLEWISYISMT
SSYTNYADSVKGRFTISRDNAKNSLSLEMNSLRVEDTAVYYCARDRLRNWNDDA
FDIWGPGTMVTVSS

SEQ ID NO: 88:

SYELTQPASVSGSPGQSITISCTGTPSDIGTYPYVSWYQQHPDRAPKLLIYGVT
NRPSGVSNRFSGSKSGNTASLTISELQAEDEADYYCSSYTLTSIVVFGGGTKLT
VLGDYKDDDDK

The antibody construct of the present invention able to bind to Phl p 6 comprises preferably SEQ ID NO: 86, SEQ ID NO: 87 and SEQ ID NO: 88 or combinations thereof.

According to a preferred embodiment of the present invention the immunoglobulin specific for Phl p 6 comprises at least one CDR sequence selected from the group consisting of SEQ ID NO: 89, SEQ ID NO: 90, SEQ ID NO: 91, SEQ ID NO: 92, SEQ ID NO: 93 and SEQ ID NO: 94. The immunoglobulin specific for Phl p 6 comprises preferably CDR sequences SEQ ID NO: 89, SEQ ID NO: 90 and SEQ ID NO: 91; and/or SEQ ID NO: 92, SEQ ID NO: 93 and SEQ ID NO: 94. The immunoglobulin specific for Phl p 6 comprises preferably a heavy chain comprising CDR sequences SEQ ID NO: 89, SEQ ID NO: 90 and SEQ ID NO: 91; and a light chain comprising CDR sequences SEQ ID NO: 92, SEQ ID NO: 93 and SEQ ID NO: 94.

According to a particularly preferred embodiment of the present invention the allergen is Bet v 1 so that the antigen binding site of the antibody construct is specific for and binds to Bet v 1.

According to a preferred embodiment of the present invention the antibody construct comprises an antigen binding site or immunoglobulin or immunoglobulin fragment specific for Bet v 1, preferably comprising an amino acid sequence selected from the group consisting of SEQ ID NO: 58, SEQ ID NO: 65 and SEQ ID NO: 66.

SEQ ID NO: 58:

MAQVQLVQSGAEVKKPGSSVKVSCKASGGTFSSYAISWVRQAPGQGLEWMGRII
PILGIVNYAQKFQGRVTITADKSTSTAYMELSSLRSEDTAVYYCAREPLGYCSG
GSCYPLHNGMDVWGQGTTVTVSSSGGGSGGGGSGGGGSDIQMTQSPDSLAVSLG
ERATINCKSSQSVLYSSNNKNYLAWYQQKPGQPPKLLFYWASTRESGVPDRFSG
SGSGTDFTLTISSLQAEDVAVYYCQQYYTTPLTFGQGTKLEIKRAAAGAPVPYP
DPLEPR

SEQ ID NO: 65:

MEFGLSWLFLVAFLKGVQCEVQLLESGGGLVQPGGSLRLSCVASGFTFTNYAMT
WVRQAPGKGLEWVSAISNYGGSTYYSDSVKGRFTISRDNSKSTLSLHMNSLRAE
DTALYYCAKYFDFWGQGTLVTVSS

SEQ ID NO: 66:

MRLPAQLLGLLLLWVPGAGCDIQLTQSPSSLSASVGDRVTITCRVSQGISNCLN
WYRRKPGKVNKVLFYSASNFQSGVPSRFSASASGTDLTLSVNTLQPEDVATCYC
QRTDNALHHFAQGTRLEIKR

The antibody construct of the present invention able to bind to Bet v 1 comprises preferably SEQ ID NO: 58 or SEQ ID NO: 65 and SEQ ID NO: 66.

According to a preferred embodiment of the present invention the immunoglobulin specific for Bet v 1 comprises at least one CDR sequence selected from the group consisting of SEQ ID NO: 59, SEQ ID NO: 60, SEQ ID NO: 61, SEQ ID NO: 62, SEQ ID NO: 63, SEQ ID NO: 64, SEQ ID NO: 67, SEQ ID NO: 68, SEQ ID NO: 69, SEQ ID NO: 70, SEQ ID NO: 16 and SEQ ID NO: 71. The immunoglobulin specific for Bet v 1 comprises preferably CDR sequences SEQ ID NO: 59, SEQ ID NO: 60 and SEQ ID NO: 61; and/or SEQ ID NO: 62, SEQ ID NO: 63 and SEQ ID NO: 64; and/or SEQ ID NO: 67, SEQ ID NO: 68 and SEQ ID NO: 69; and/or SEQ ID NO: 70, SEQ ID NO: 16 and SEQ ID NO: 71. The immunoglobulin specific for Bet v 1 comprises preferably a heavy chain comprising CDR sequences SEQ ID NO: 59, SEQ ID NO: 60 and SEQ ID NO: 61; and a light chain comprising CDR sequences SEQ ID NO: 62, SEQ ID NO: 63 and SEQ ID NO: 64; or a heavy chain comprising CDR sequences SEQ ID NO: 67, SEQ ID NO: 68 and SEQ ID NO: 69; and a light chain comprising CDR sequences SEQ ID NO: 70, SEQ ID NO: 16 and SEQ ID NO: 71.

**Table A:**

| **SEQ ID NO:** | **Sequence** | **CDR** |
|---|---|---|
| 8 | GGSIRSGGYY | CDR1 |
| 9 | IYHSGNT | CDR2 |
| 10 | ARLDGYTLDIW | CDR3 |
| 11 | QRINTY | CDR1 |
| 12 | AAS | CDR2 |
| 13 | QESLSASYT | CDR3 |
| 14 | ARSDGYTLDN | CDR3 |
| 15 | QSISTY | CDR1 |
| 16 | SAS | CDR2 |
| 17 | QQSYTTLYT | CDR3 |
| 18 | QGISSY | CDR1 |
| 19 | QQANSFPYT | CDR3 |
| 30 | GFTFDDHA | CDR1 |
| 31 | VSWNSGTR | CDR2 |
| 32 | ARDMRAATPYSFDY | CDR3 |
| 33 | QTFNNY | CDR1 |
| 34 | QQSYSTPLT | CDR3 |
| 35 | ISWNSGVV | CDR2 |
| 36 | VRDFRAASPYFFDY | CDR3 |
| 37 | QSIGNY | CDR1 |
| 38 | QQSNRTPIT | CDR2 |
| 39 | GLTFDDYA | CDR1 |
| 40 | ISWNSGVR | CDR2 |
| 41 | AKDIRAATPYALDH | CDR3 |
| 42 | RTIYNY | CDR1 |
| 43 | QQSHGTPLT | CDR3 |
| 44 | EFTFNNYW | CDR1 |
| 45 | IKPDGSVK | CDR2 |
| 46 | ARERQSGAFGL | CDR3 |
| 47 | QSISSY | CDR1 |
| 48 | GFTFDDYA | CDR1 |
| 49 | ISWNAGSI | CDR2 |
| 50 | AREWDGTAYYGYTVGY | CDR3 |
| 51 | QSLVYSDGNTY | CDR1 |
| 52 | KVS | CDR2 |
| 53 | MQGTHWPPT | CDR3 |
| 54 | GFTFSSYA | CDR1 |
| 55 | ISGSGGST | CDR2 |
| 56 | ARVLRNQAAAGTIRWEDP | CDR3 |
| 57 | QSLVYSDGNT | CDR1 |
| 59 | GGTFSSYA | CDR1 |
| 60 | IIPILGIV | CDR2 |
| 61 | AREPLGYCSGGSCYPLHNGMDV | CDR3 |
| 62 | QSVLYSSNNKNY | CDR1 |
| 63 | WAS | CDR2 |
| 64 | QQYYTTPLT | CDR3 |
| 67 | GFTFTNYA | CDR1 |
| 68 | ISNYGGST | CDR2 |
| 69 | AKYFDF | CDR3 |
| 70 | QGISNC | CDR1 |
| 71 | QRTDNALHH | CDR3 |
| 77 | EFAFDSYE | CDR1 |
| 78 | ISIDETNK | CDR2 |
| 79 | ARWNNWNHRGMVFAFDL | CDR3 |
| 80 | QGISSW | CDR1 |
| 81 | HSISNY | CDR1 |
| 82 | QESFSPSGT | CDR3 |
| 83 | QSILGY | CDR1 |
| 84 | QQSYITPRT | CDR3 |
| 85 | QQSHSTPYT | CDR3 |
| 89 | GFIFSDHY | CDR1 |
| 90 | ISMTSSYT | CDR2 |
| 91 | ARDRLRNWNDDAFDI | CDR3 |
| 92 | PSDIGTYPY | CDR1 |
| 93 | GVT | CDR2 |
| 94 | SSYTLTSIVV | CDR3 |

According to another preferred embodiment of the present invention the allergen is a mite allergen, preferably Der p 1, Der p 2, Der p 3, Der p 4, Der p 5, Der p 6, Der p 7, Der p 8, Der p 9, Der p 10, Der p 11, Der p 14, Der p 15, Der p 18, Der p 20, Der p 21 and Der p 23.

According to a further preferred embodiment of the present invention the allergen is an animal, allergen, selected from a group consisting of a cat allergen, preferably Fel d 1, Fel d 2, Fel d 3, Fel d 4, Fel d 5, Fel d 6, Fel d 7 and Fel d 8, a dog allergen, preferably Can f 1, Can f 2, Can f 3, Can f 4 and Can f 5, and a cockroach allergen, preferably Bla g 1, Bla g 2, Blag 3, Blag 4, Blag 5, Blag 6, Bla g 7, Bla g 8, Bla g 11, Per a 1, Per a 2, Per a 3, Per a 6, Per a 7, Per a 9 and Per a 10.

According to preferred embodiment of the present invention the immunoglobulin specific for ICAM1 is an ICAM1-specific human or humanized mammalian, preferably mouse, IgG₁ antibody.

The antigen binding site specific for or binding to ICAM1 can be part of an immunoglobulin which has been obtained by methods known in the art. Particularly preferred the immunoglobulin or immunoglobulin fragment comprising said antigen binding site is an IgG, more preferably an IgG₁, antibody or fragment thereof.

Another aspect of the present invention relates to a pharmaceutical composition comprising the antibody construct of the present invention.

The antibody construct of the present invention can be provided in a pharmaceutical composition which may comprise next to at least one antibody construct of the present invention further ingredients such as other active compounds and/or at least one pharmaceutically acceptable excipient.

In a particularly preferred embodiment the pharmaceutical composition of the present invention comprises an antibody construct as defined above which comprises an antigen binding site or immunoglobulin or immunoglobulin fragment specific for Bet v 1 and/or for Phl p 1, Phl p 2, Phl p 5, Phl p 6 or combinations thereof.

"Pharmaceutically acceptable excipient" as used herein refers to a carrier, usually a liquid, in which an active therapeutic agent is formulated. The excipient generally does not provide any pharmacological activity to the formulation, though it may provide chemical and/or biological stability, and release characteristics. Exemplary formulations can be found, for example, in Remington's Pharmaceutical Sciences, 19th Ed., Grennaro, A., Ed., 1995. The term includes further any and all solvents, dispersion media, coatings, antibacterial and antifungal agents, isotonic and absorption delaying agents that are physiologically compatible. In one embodiment, the carrier is suitable for intranasal, oral or ocular administration or for inhalation. Pharmaceutically acceptable carriers and excipients include sterile aqueous solutions or dispersions and sterile powders. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Pharmaceutical compositions typically must be sterile and stable under the conditions of manufacture and storage. The invention contemplates that the pharmaceutical composition is present in lyophilized form. The composition can be formulated as a solution, microemulsion, liposome, or other ordered structure. The earner can be a solvent or dispersion medium containing, for example, water, polyol (for example, glycerol, propylene glycol, and liquid polyethylene glycol), and suitable mixtures thereof. The invention further contemplates the inclusion of a stabilizer in the pharmaceutical composition. The proper fluidity can be maintained, for example, by the use of a coating such as lecithin, by the maintenance of the required particle size in the case of dispersion and by the use of surfactants.

The pharmaceutical composition of the present invention comprises preferably isotonic agents such as, for example, sodium chloride. For each of the recited embodiments, the compounds can be administered by a variety of dosage forms. Any biologically-acceptable dosage form known to persons skilled in the art, and combinations thereof, are contemplated. Examples of such dosage forms include reconstitutable powders, liquids, solutions, suspensions, emulsions, powders, granules, particles, microparticles, cachets, inhalants, aerosol inhalants, particle inhalants, and combinations thereof.

According to a preferred embodiment of the present invention the composition is adapted for intranasal, oral or ocular administration or for inhalation.

According to a further preferred embodiment of the present invention the pharmaceutical composition of the present invention is used for treating and/or preventing an allergy.

According to another preferred embodiment of the present invention the pharmaceutical composition of the present invention is used for desensitizing an allergic patient or a patient at risk to develop an allergy.

The pharmaceutical composition of the present invention cannot only be used in the treatment and in the prevention of an allergy.

According to a preferred embodiment of the present invention the pharmaceutical composition of the present invention is used for preventing, reducing and/or alleviating allergic symptoms in a patient suffering from an allergy or allergic symptoms.

According to a further preferred embodiment of the present invention the pharmaceutical composition of the present invention comprises 0.05 to 1000 µg, preferably 5 to 1000 µg, more preferably 50 to 1000 µg, even more preferably 100 to 1000 µg, of the antibody construct of the present invention.

Administration of the antibody construct and the pharmaceutical preparations according to the present invention to an individual in need thereof can be carried out using known procedures at dosages and for periods required. Effective amounts of the therapeutic compositions will vary according to factors such as the age, sex and weight of the individual and the ability of the protein or fragment thereof to elicit an antigenic response in the individual. The antibody construct and the pharmaceutical preparations may be administered in a convenient manner such as by intranasal, oral or ocular administration or by inhalation, whereby for inhalation allergens the administration by inhalation is most preferred. For example, about 0.05 to 1000 µg, more preferably from about 0.1 to 100µg, even more preferably 0.5 to 100µg, of active compound (i.e. antibody construct) per dosage unit may be administered. Dosage regimen may be adjusted to provide the optimum therapeutic response. For example, several divided doses may be administered daily or the dose may be proportionally reduced as indicated by the exigencies of the therapeutic situation. The pharmaceutical preparation of the present invention can be administered, for instance, for 1 to 6 times before an allergy season at intervals of 1 to 5 weeks and/or during the allergy season. During an allergy season the pharmaceutical preparation of the present invention is preferably administered at intervals in between 6 and 96 hours, preferably between 12 and 48 hours. If the allergen is a food allergen, the antibody construct of the present invention can be administered before or during the uptake of the respective food. The administration occurs preferably 1 to 60 minutes, more preferably 2 to 40 minutes, even more preferably 5 to 30 minutes, before the allergen comprising food is consumed.

The terms "preventing" and "prevention", as used herein, refer to the prevention or inhibition of the recurrence, onset and development of an allergy or a symptom thereof in a subject resulting from the administration of the fusion protein or pharmaceutical preparation according to the present invention. In some embodiments "preventing" and "prevention" refers to the reduction of the risk to develop an allergy against specific allergens. The term "preventing" covers measures not only to prevent the occurrence of an allergy, but also to arrest its progress and reduce its consequences once established.

The terms "treatment" and "treating", as used herein, refer to the reduction or inhibition of the progression and duration of an allergy, the reduction or amelioration of the severity of the allergy and the amelioration of one or more symptoms thereof. "Treatment" encompasses also the improvement and/or reversal of the symptoms of an allergy or allergic reactions. A fusion protein which causes an improvement in any parameter associated with allergy may be identified as a therapeutic protein. The term "treatment" refers to both therapeutic treatment and prophylactic measures. For example, those who may benefit from treatment with compositions and methods of the present invention include those already with an allergy as well as those in which the allergy is to be prevented.

A further aspect of the present invention relates to a method of treating and/or preventing an allergy, or preventing, reducing and/or alleviating allergic symptoms in a patient suffering from an allergy or allergic symptoms. In a particularly preferred embodiment birch pollen and grass pollen allergy are treated with an antibody construct as defined above which comprise an antigen binding site or immunoglobulin or immunoglobulin fragment specific for Bet v 1 and/or for Phl p 1, Phl p 2, Phl p 5, Phl p 6 or combinations thereof.

The present invention is further illustrated in the following figures and examples, however, without being restricted thereto.

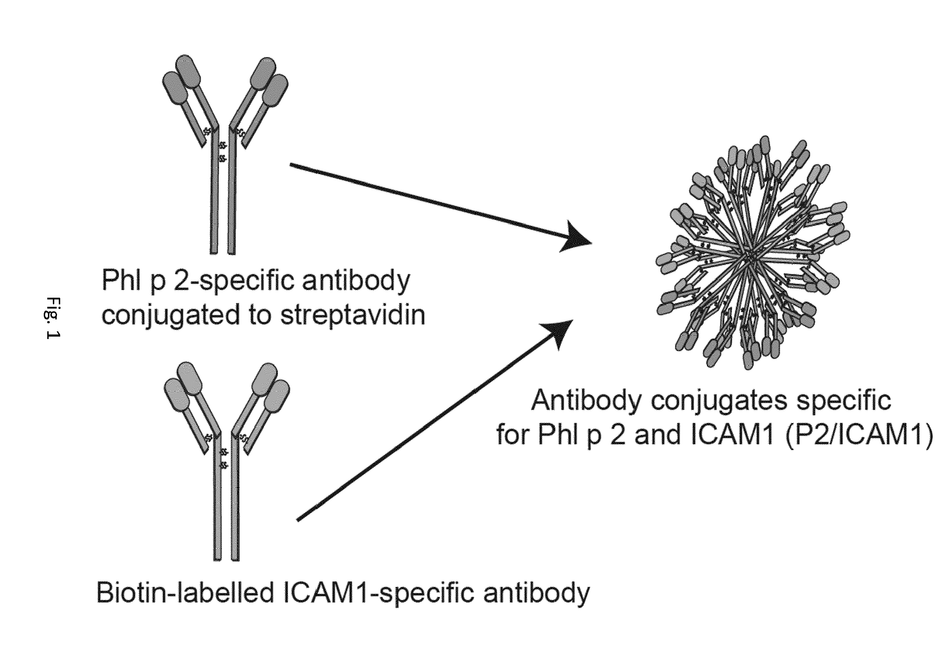

**FIG 1****.** Generation of antibody conjugates containing Phl p 2-and ICAM1-specific antibodies. A Phl p 2-specific monoclonal human IgG₁ antibody was conjugated to streptavidin and subsequently conjugates, termed P2/ICAM1 were formed with a biotin-conjugated ICAM1-specific mouse monoclonal antibody.

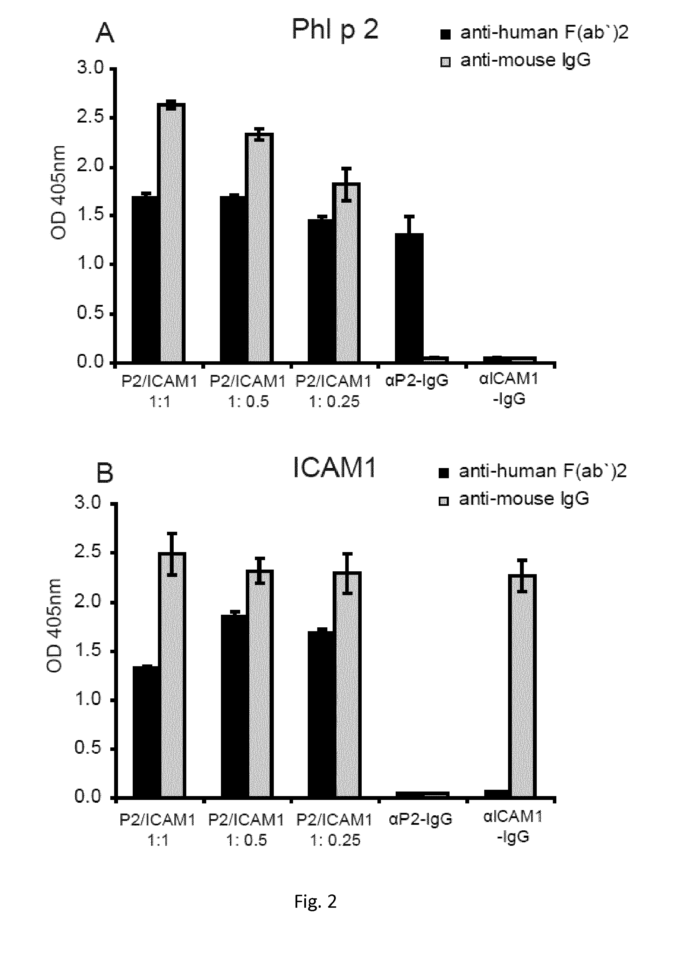

**FIG 2****.** Reactivity of P2/ICAM1 with Phl p 2 and ICAM1. Conjugates formed with different ratios of anti-Phl p 2 and anti-ICAM1 (x-axes: P2/ICAM1: 1:1; 1:0.5; 1:025), anti-Phl p 2 (αP2-IgG) or anti-ICAM1 (αICAM1-IgG) alone were tested for reactivity with Phl p 2 **(A)** or ICAM1 **(B)** and detected with anti-human F(ab')₂ antibodies (black bars) or anti-mouse IgG (gray bars). OD values (y-axes) corresponding to bound P2/ICAM1 conjugates are shown as means of triplicates +/- SD.

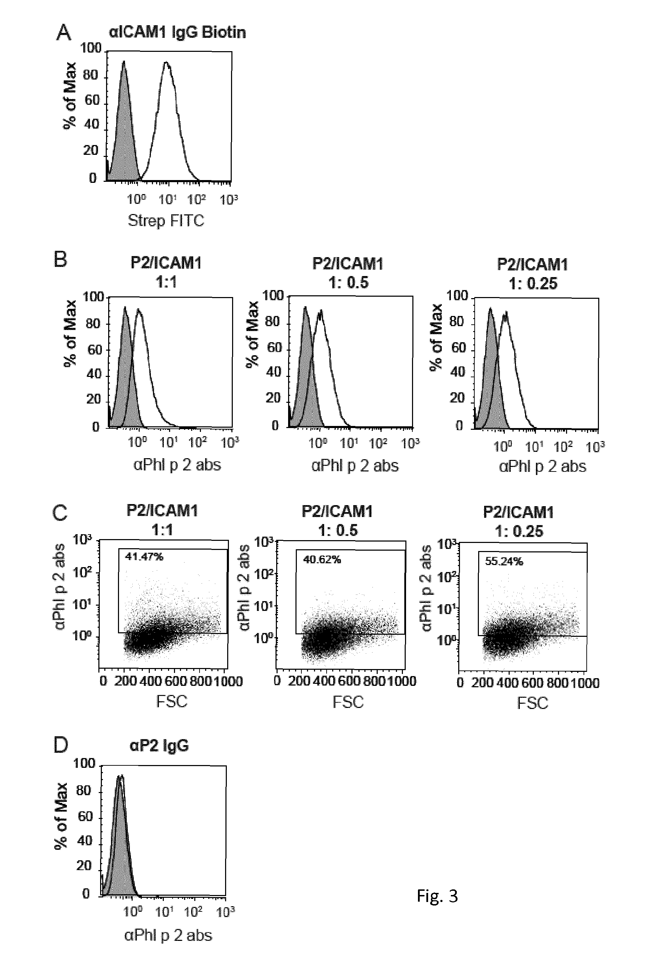

**FIG 3****.** Detection of ICAM1 or P2/ICAM1-captured Phl p 2 on respiratory epithelial cells using flow cytometry. **(A)** ICAM1 was detected on the surface of 16HBE14o- cells using ICAM1-specific antibodies (white graph) or an isotype control (gray graph). **(B)** Cells pre-incubated with different ratios of P2/ICAM1 (1:1; 1:0.5; 1:0.25) and Phl l p 2 were probed for cell-bound Phl p 2 with specific rabbit antibodies (white graphs) or the isotype control (gray graph). **(C)** Scatterplots showing the percentages of Phl p 2-reactive cells with three different ratios of P2/ICAM1. **(D)** Cells incubated with Phl p 2-specific human IgG₁ antibodies followed by Phl p 2 and Phl p 2-specific rabbit antibodies. Shown is one representative out of three independent experiments.

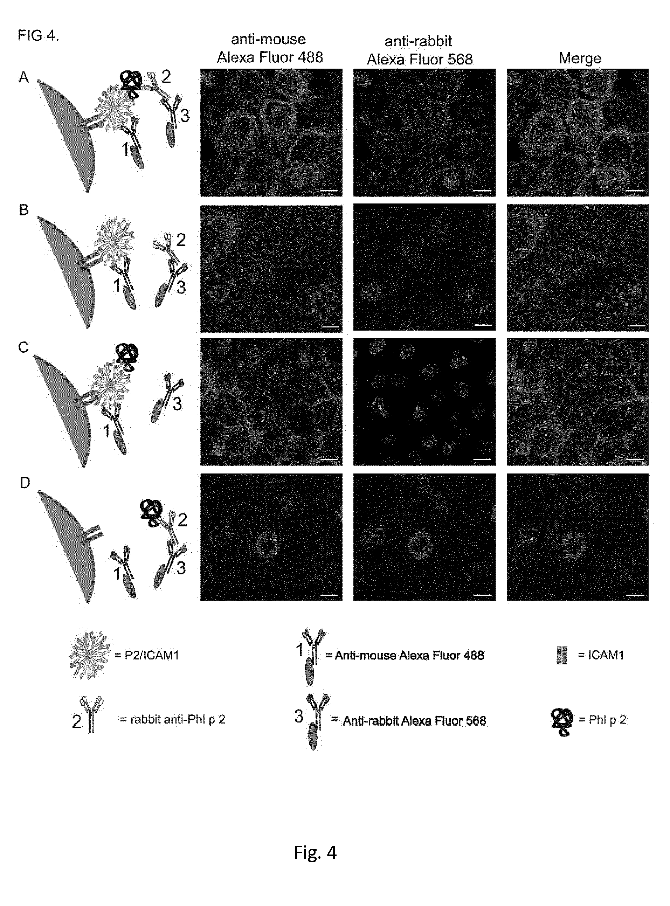

**FIG 4****.** Visualization of P2/ICAM1 and Phl p 2 on 16HBE14o-cells by immunofluorescence microscopy. Images showing cells incubated with different combinations of reactants (left margin and bottom), which were stained with Alexa Fluor®488-labelled anti-mouse antibodies and Alexa Fluor®568-labelled anti-rabbit antibodies to visualize αICAM1-IgG and Phl p 2, respectively. Nuclei were stained with DAPI and merged images are shown in the right column. Bars in the images represent 20 µm.

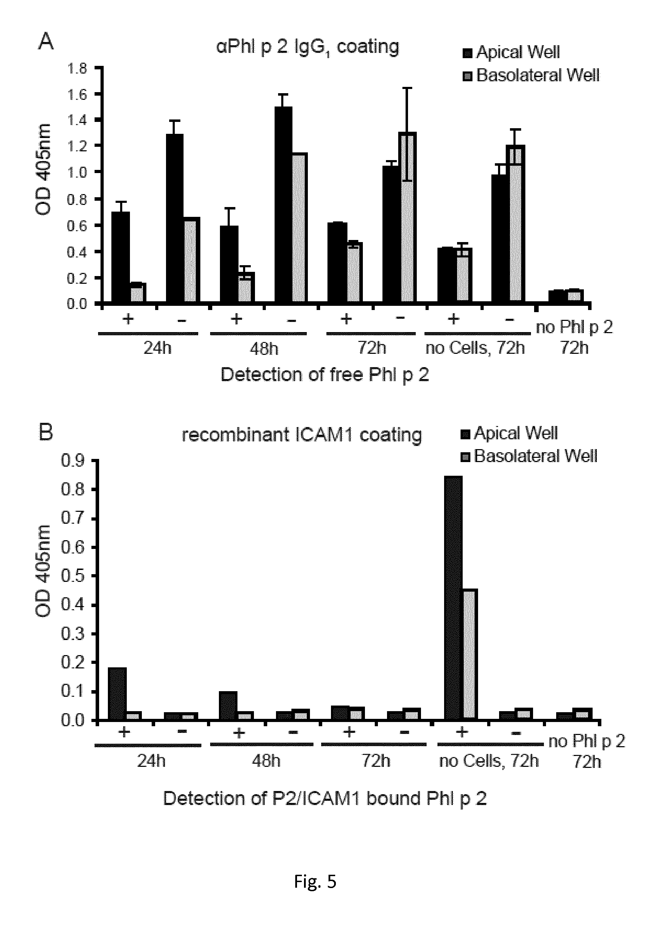

**FIG 5****.** Cell-bound P2/ICAM1 conjugates inhibit trans-epithelial migration of Phl p 2. **(A)** Detection of free Phl p 2 in apical (black bars) and basolateral (gray bars) compartments of cultured 16HBE14o- cell monolayers which had been pre-incubated with (+) or without (-) P2/ICAM1 conjugates. Concentrations of free Phl p 2 corresponding to OD values (y-axis) were measured at different points of time (x-axis: 24h, 48h, 72h) in wells with or without cells (no cells). In the negative control, Phl p 2 was omitted (no Phl p 2). **(B)** Simultaneous detection of P2/ICAM1-Phl p 2 complexes in the very same samples as in **(A).** One out of three independent experiments giving comparable results is shown.

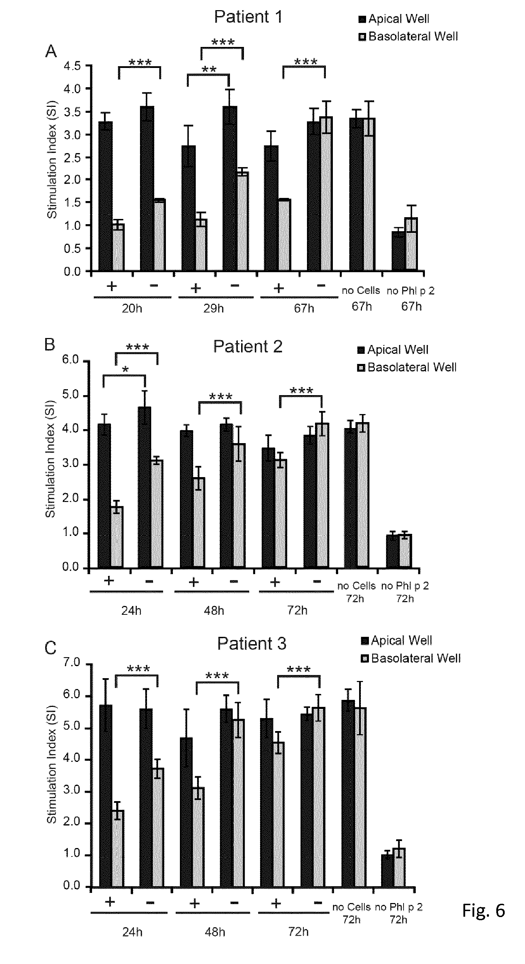

**FIG. 6****.** Inhibition of trans-epithelial migration of Phl p 2 by P2/ICAM1 conjugates leads to decreased basophil activation with basolateral samples. Samples obtained from apical and basolateral compartments of cultures treated as in Fig. 5 were incubated with blood samples from three Phl p 2 allergic patients **(A-C)** and basophil activation was measured by determining up-regulation of the surface marker CD203c on basophils by flow cytometry. CD203c up-regulation expressed as stimulation index (SI) is displayed on the y-axis. Results are means of triplicates and error bars indicate standard deviation. *P < .05, ** < .01, and *** P < .001, ANOVA and linear contrasts.

### EXAMPLES:

### Example 1: P2/ICAM1 antibody conjugates specifically bind Phl p 2 and ICAM1

### Antibodies and antibody conjugation

A Phl p 2-specific IgE Fab-fragment was previously isolated from an allergic patient as described in Steinberger et al. (J Biol Chem 271(1996):10967-10972). This Fab was converted into a fully human IgG₁ antibody and stably expressed in CHO-K1 cells. This Phl p 2-specific IgG₁ was purified via Protein G affinity chromatography (Thermo Fisher Scientific, Pierce, USA). The Phl p 2-specific IgG₁ antibody was conjugated with Lightning-Link® Streptavidin (Innova Biosciences, UK). A biotin-labeled ICAM1-specific mouse IgG₁ antibody was purchased from Abcam (UK). To form bi-specific antibody conjugates, termed P2/ICAM1, three different ratios (1:1, 1:0.5, 1:0.25) of the Phl p 2-specific human IgG₁ and the ICAM1-specific mouse IgG₁ were co-incubated for at least one hour at room temperature.

### ELISA evaluation of the binding specificities of P2/ICAM1 conjugates

ELISA plates (Nunc Maxi-Sorp, Roskilde, Denmark) were coated with recombinant ICAM1 (R&D Systems, Minneapolis, USA) or recombinant Phl p 2 (Biomay AG, Austria) (5 µg/ml in 100mM Na-HCO₃, pH = 9.6). Plates were incubated for 1h at 37°C, washed twice with phosphate-buffered saline (PBS) containing 0.05% (v/v) Tween-20 (PBST) and saturated with PBST containing 3% (w/v) bovine serum albumin (BSA). P2/ICAM1 (1 µg/ml) was applied overnight at 4°C. Bound P2/ICAM1 was detected either with horseradish peroxidase-conjugated rat anti-mouse IgG₁ antibodies (BD PharMingen, San Diego, CA, USA) or with alkaline phosphatase-conjugated goat anti-human F(ab')₂ antibodies (Pierce), both diluted 1:5000 in PBST containing 1% (w/v) BSA. Optical density (OD) measurements were carried out on ELISA reader Spectramax Plus (Molecular Devices, Sunnyvale, CA, USA) at 405 nm. Results are means of triplicates. Error bars indicate standard deviations.

### Results:

Antibody conjugates, termed P2/ICAM1 consisting of a Phl p 2- (i.e., P2) and an ICAM1-specific monoclonal antibody (i.e., ICAM1) were formed through streptavidin-biotin coupling (i.e., conjugation) using different ratios of the individual components (P2/ICAM1: 1:1, 1:0.5, 1:0.25) (Fig 1). In a first series of experiments it was investigated if formed antibody conjugates retain binding specificity for Phl p 2 and ICAM1 (Fig 2). Conjugates consisting of different ratios of the monoclonal antibodies showed comparable binding to ELISA plate-bound Phl p 2 with the non-conjugated anti-Phl p 2 antibody when detected with anti-human F(ab')₂ indicating that the latter was fully functional in the conjugate (Fig 2 A). Phl p 2-bound conjugates could be also detected with the anti-mouse IgG antibody with decreasing intensities reflecting the proportion of the ICAM1-specific antibody in the conjugate (Fig 2 A). No binding to Phl p 2 was detected with anti-mouse IgG antibodies when non-conjugated Phl p 2-specific antibodies were tested or when anti-ICAM1 antibodies were tested with coated Phl p 2 (Fig 2 A). Binding of conjugate to ICAM1 could be demonstrated by detecting the Phl p 2-specific antibody as well as the ICAM1-specific antibody (Fig 2 *B*)*.* The intensities of binding with the conjugates were similar as for the non-conjugated ICAM1-specific antibody. No binding of the non-conjugated ICAM1-specific antibody to ICAM1 was found with anti-human F(ab')₂. Also no reactivity of the non-conjugated Phl p 2-specific antibody to ICAM1 was observed with any of the detection systems (Fig 2 *B*)*.*

### Example 2: Phl p 2 can be immobilized via P2/ICAM1 onto respiratory epithelial cells

### Flow cytometry

The epithelial cell line 16HBE14o- was derived from human bronchial surface epithelial cells and cultivated. Aliquots of approximately 150.000 16HBE14o- cells were incubated with 1 µg of P2/ICAM1 in 50 µl FACS buffer (PBS supplemented with 2% w/v BSA) for 30 min on ice. After washing, aliquots of 1 µg Phl p 2 in 50 µl FACS buffer were added and cells were again incubated for 30 min on ice. After further washing, cells were incubated with Phl p 2-specific rabbit antibodies and an Alexa Fluor®488-labelled goat anti-rabbit antibody (Molecular Probes, Life Technologies, Eugene, Oregon, US). Biotin conjugated anti-ICAM1 antibody was visualized with Streptavidin conjugated to Alexa Fluor®488 (Molecular Probes). 7-AAD-dye (Beckman Coulter, Brea, California, US) was used for staining of dead cells. Controls included the biotin conjugated anti-ICAM1 antibody isotype control (mouse IgG₁ MOPC-21, Abcam) or the Phl p 2-specific antibody conjugated to Streptavidin followed by Phl p 2 and Phl p 2-specific rabbit antibodies. Cells were analysed on a FC500 Flow Cytometer (Beckman-Coulter) counting at least 30.000 cells per sample in duplicates and were evaluated with FlowJo version 7.2.5 (Treestar, Ashland, Oregon, US).

### Immunofluorescence Microscopy

16HBE14o- cells, which had been cultured as described above were seeded on ibiTreat tissue culture-treated µ-dishes (Ibidi GmbH, Munich, Germany) and grown for 2 days to approximately 50% confluence. Cells were washed twice with PBS, cooled to 4°C and further processed at 4°C unless stated otherwise. Antibody-allergen complexes were formed by co-incubation of 5 µg P2/ICAM1 and 1 µg Phl p 2 in 300 µl PBS for 30 min at room temperature and subsequently incubated with the cells at 4°C for 30 min. Unbound complexes were removed by washing with PBS and cells were then incubated with Phl p 2-specific rabbit antibodies diluted 1:1000 in PBS. Cells were washed with PBS, fixed with 4% formaldehyde solution for 20 min at room temperature and remaining aldehyde groups were quenched with 50mM ammonium chloride in PBS for 10 min. Unspecific binding sites were blocked with 10% goat serum in PBS overnight at 4°C. To detect cell-bound P2/ICAM1, Alexa Fluor® 488 goat anti-mouse IgG (Molecular Probes) diluted 1:1000 in PBS containing 10% goat serum was added to the cells for 1 hour at room temperature. Phl p 2 bound to P2/ICAM1 was visualized with Alexa Fluor® 568 goat anti-rabbit IgG (Molecular Probes) applied at a dilution of 1:1000 in PBS for 1 hour at room temperature. Finally, nuclei were stained with 1ug/ml DAPI (Molecular Probes) in PBS. Cells were washed twice with PBS between individual incubations. Staining with fluorescent antibodies alone was done to assess background signals. Controls omitting either Phl p 2, Phl p 2-specific rabbit antibodies or P2/ICAM1 were included. Wide-field fluorescence imaging of fixed cells was carried out using a Zeiss Axio Observer Z1 inverted fluorescence microscope equipped with an oil immersion 40×lens (Pan Apochromat, 1.4 NA, Carl Zeiss Inc., Oberkochen, Germany) and the Zeiss AxioVision Software package (Release 4.8).

### Results

To study if P2/ICAM1 can be used to immobilize Phl p 2 on the surface of respiratory epithelial cells, 16HBE14o- cells, which express ICAM1 on their surface as shown by FACS analysis (Fig 3 A), were used as surrogate. Cells were first incubated with P2/ICAM1 conjugates (different ratios of P2/ICAM1: 1:1, 1:0.5, 1:0.25) and, after washing, exposed to Phl p 2. Using Phl p 2-specific rabbit antibodies immobilization of the Phl p 2 allergen onto the cell surface via P2/ICAM1 could be demonstrated (Fig 3 B). A scatter plot analysis showed that immobilization was best (i.e., more than 50% of the cells had Phl p 2 bound to their cell surface) when the ratio of Phl p 2-specific antibody to ICAM1-specific antibody was 1:0.25 (Fig 3 C). No staining of cells was observed when the non-conjugated Phl p 2-specific antibody followed by Phl p 2 was added to cells and exposed to Phl p 2-specific rabbit antibodies (Fig 3 *D*)*.*

To study the immobilization of Phl p 2 via P2/ICAM1 on the cells in more depth, immunofluorescence microscopy experiments were conducted. Images showed co-localization (Fig. 4A, merge) of Phl p 2 (Alexa Fluor®568) and P2/ICAM1 (Alexa Fluor®488) on the cell surface. When either Phl p 2 (Fig 4 B) or Phl p 2-specific rabbit antibodies (Fig 4 C) were omitted, no binding of Alexa Fluor® 568 goat anti-rabbit IgG was observed. Whenever P2/ICAM1 was added to the cells it was detected with Alexa Fluor 488 goat anti-mouse IgG specific for αICAM 1 IgG (Fig 4A, *B,* C). When P2/ICAM1 was omitted, detection antibodies did not bind (Fig 4 *D*)*.* No staining of cells was found when only secondary antibodies alone were applied.

### Example 3: P2/ICAM1 conjugates prevent migration of Phl p 2 through respiratory epithelial cell layers

### ELISA differentiating free Phl p 2 from P2/ICAM1-bound Phl p 2 in culture samples

To measure free Phl p 2 in culture samples the Phl p 2-specific human IgG₁ was coated (1 µg/ml in 100mM NaHCO₃, pH = 9.6) to ELISA plates, which were washed and blocked as described above. Phl p 2 was then detected with rabbit anti-Phl p 2 antibodies (1:1000). Bound rabbit antibodies were visualized with a horseradish peroxidase-conjugated donkey anti-rabbit antibody diluted 1:2000 (GE Healthcare, Little Chalfont, UK).

To detect P2/ICAM1-bound Phl p 2, recombinant ICAM1 (1 µg/ml in 100mM NaHCO₃, pH = 9.6) was coated to ELISA plates, which were washed and blocked as describe above. Cell culture supernatants were applied and P2/ICAM1-bound Phl p 2 was detected with rabbit anti-Phl p 2 antibodies as describe above. Results represent means of duplicates with error bars indicating standard deviation.

### Cell culture and transwell experiments

The epithelial cell line 16HBE14o- was derived from human bronchial surface epithelial cellsand cultivated. TER baseline ranged consistently from 110 to 120Ω/cm² for transwells filled with medium without cells and was measured with an ohm voltmeter (Millipore, USA) When cells reached a TER value of 400Ω/cm² (baseline subtracted) IFN-γ (50ng/ml, Pepro Tech Inc; USA) was added in all cultures to allow detectable allergen migration. Aliquots of 5 µg P2/ICAM1 (ratio 1:0.25) were applied to the apical chambers for three hours at 37°C. Supernatants were removed and 10ng Phl p 2 in 0.5ml medium was added to the apical compartment for 20h-72h at 37°C. The effect of P2/ICAM1 on Phl p 2 apical-to-basolateral trans-epithelial migration was assessed by comparing P2/ICAM1 treated wells with untreated control wells. In addition, the transmigration of Phl p 2 as well as of P2/ICAM1-bound Phl p 2 through transwell membranes without cells was assessed. Samples from apical and basolateral compartments were tested for the presence of free Phl p 2 and/or P2/ICAM1-bound Phl p 2 by ELISA.

### Results:

In a next series of experiments it was investigated whether P2/ICAM1 can prevent the apical-to-basolateral penetration of Phl p 2 through a layer of cultured respiratory epithelial cells. For this purpose, the apical compartments of cells were pre-incubated with or without P2/ICAM1 conjugates. Subsequently the Phl p 2 allergen was added to the apical side and its penetration through the layer into the basolateral compartment was measured with a catching ELISA differentiating free Phl p 2 from P2/ICAM1-bound Phl p 2 (Fig 5A, *B*). The apical addition of P2/ICAM1 strongly prevented the penetration of Phl p 2 over the full period of analysis (i.e., for 72 hours) into the basolateral compartment (see Fig 5A, gray bars +) when compared to conditions without addition of P2/ICAM1 (Fig 5A, gray bars -). When Phl p 2 and P2/ICAM1 conjugates were omitted, no Phl p 2 signal was detected (Fig 5A, no Phl p 2, 72h).

In this experimental set up the apical-to-basolateral penetration of the complex between P2/ICAM1 and the Phl p 2 allergen was also studied (Fig 5 *B*). This was achieved with a sandwich ELISA using recombinant ICAM1 for catching the P2/ICAM1 conjugates and detecting bound Phl p 2 with rabbit anti-Phl p 2 antibodies. In fact, when cell monolayers were used no relevant penetration of P2/ICAM1-Phl p 2 complexes into the basolateral wells was found (Fig 5 *B*). Therefore, we also performed experiments without cells where P2/ICAM1 and Phl p 2 was added simultaneously showing that P2/ICAM1-Phl p 2 immune complexes were formed and penetrated into the basolateral well when no cells were present (Fig 5 *B*, no cells, 72h).

### Example 4: Prevention of trans-epithelial allergen migration by P2/ICAM1 leads to decreased basophil activation

### Basophil activation assays

Basophil activation was assessed *in vitro* by measuring up-regulation of CD203c expression. Heparinized blood from grass pollen allergic patients with IgE antibodies against Phl p 2 were obtained after informed consent was given. Blood aliquots (90µl) were incubated with either samples from the transwell experiments or for control purposes with a monoclonal anti-IgE antibody (1 mg/mL; Immunotech, Vaudreuil-Dorion, Quebec, Canada) or PBS for 15 minutes at 37°C. Allergen-induced up-regulation of CD203c was calculated from mean fluorescence intensities (MFIs) obtained with stimulated (MFIstim) and unstimulated (MFIcontrol) cells and expressed as stimulation index (SI) (MFIstim /MFIcontrol). Cells were analyzed by 2-color flow cytometry on a FACScan (BD Biosciences, USA).

### Results

To test whether a reduction of trans-epithelial allergen migration by P2/ICAM1 has an effect on basophil activation basophil activation experiments using samples from the apical and basolateral compartments taken from three independent transwell experiments were performed. Results from these experiments with samples of three representative allergic patients are shown in Fig 6 *A*, *B, C*. Comparing the extent of basophil activation induced with basolateral samples showed a significant and consistent reduction of basophil activation for samples taken from cultures where P2/ICAM1 had been added to the apical compartments (Fig 6 *A*, *B, C*, gray bars +) compared to those where P2/ICAM1 conjugates had been omitted (gray bars -). This effect was observed at each of the analyzed time points. The extent of basophil-activation by samples taken from the apical compartments (black bars) was comparable and almost identical to that obtained with basolateral samples from cell-free preparations (Fig 6 *A*, *B, C*, no cells). Basophil activation was not observed when Phl p 2 was absent from the cultures (Fig 6 *A*, *B, C*, no Phl p 2).

## Claims

1. An antibody construct comprising at least one antigen binding site specific for ICAM1 and at least one antigen binding site specific for at least one allergen.

2. The antibody construct of claim 1, wherein the antibody construct is a complex, conjugate or fusion protein comprising an antigen binding site specific for ICAM1 and an antigen binding site specific for an allergen.

3. The antibody construct of claim 1 or 2 comprising an antigen binding site specific for ICAM1 chemically cross-linked to an antigen binding site specific for an allergen.

4. The antibody construct of claim 1, wherein the antibody construct is a bispecific antibody, a bispecific diabody or a bispecific (Fab)₂ fragment.

5. The antibody construct of any one of claims 1 to 4, wherein the allergen is an inhalation or food allergen.

6. The antibody construct of claim 5, wherein the inhalation allergen is selected from the group consisting of pollen allergens, mold allergens, mite allergens and animal allergens, preferably pet allergens.

7. The antibody construct of any one of claims 1 to 5, wherein the allergen is selected from the group consisting of a pollen allergen, mite allergen, cat allergen, dog allergen, insect bite/sting allergen, legume allergen, fruit allergen, nut allergen, milk allergen and fish allergen.

8. The antibody construct of any one of claims 1 to 7, wherein the allergen is a pollen allergen selected from the group of a grass pollen allergen, preferably the grass pollen allergen Phl p 1, Phl p 2, Phl p 4, Phl p 5, Phl p 6, Phl p 7, Phl p 11, Phl p 12 or Phl p 13, a weed allergen, preferably the ragweed allergen Amb a 1, Amb a 2, Amb a 3, Amb a 4, Amb a 5, Amb a 6, Amb a 7, Amb a 8, Amb a 9 or Amb a 10, a mugwort allergen, preferably Art v 1, Art v 2, Art v 3, Art v 4, Art v 5 or Art v 6, a Pari-taria pollen allergen, preferably Par j 1, Par j 2, Par j 3 or Par j 4, a olive pollen allergen, preferably Ole e 1, Ole e 2, Ole e 3, Ole e 4, Ole e 5, Ole e 6, Ole e 7, Ole e 8, Ole e 9, Ole e 10 or Ole e 11, a pollen allergen from Saltwort thistle, preferably Sal k 1, Sal k 2, Sal k 3, Sal k 4 or Sal k 5, and a tree pollen, preferably a Cypress pollen, preferably Cha o 1 or Cha o 2, or a cedar pollen, preferably Jun a 1, Jun a 2 or Jun a 3, or a birch pollen allergen, preferably Bet v 1.

9. The antibody construct of any one of claims 1 to 8, wherein the allergen is Phl p 2.

10. The antibody construct of any one of claims 1 to 7, wherein the allergen is a mite allergen, preferably Der p 1, Der p 2, Der p 3, Der p 4, Der p 5, Der p 6, Der p 7, Der p 8, Der p 9, Der p 10, Der p 11, Der p 14, Der p 15, Der p 18, Der p 20, Der p 21 and Der p 23.

11. The antibody construct of any one of claims 1 to 7, wherein the allergen is an animal, preferably pet allergen, selected from a group consisting of a cat allergen, preferably Fel d 1, Fel d 2, Fel d 3, Fel d 4, Fel d 5, Fel d 6, Fel d 7 and Fel d 8, a dog allergen, preferably Can f 1, Can f 2, Can f 3, Can f 4 and Can f 5, and a cockroach allergen, preferably Bla g 1, Bla g 2, Bla g 3, Bla g 4, Bla g 5, Blag 6, Blag 7, Bla g 8, Bla g 11, Per a 1, Per a 2, Per a 3, Per a 4, Per a 5, Per a 6, Per a 7, Per a 9 and Per a 10.

12. The antibody construct of any one of claims 1 to 11, wherein the antibody construct comprises at least one immunoglobulin or at least one immunoglobulin fragment comprising an antigen binding site for ICAM1 and at least one immunoglobulin or at least one immunoglobulin fragment comprising an antigen binding site for at least one allergen.

13. A pharmaceutical composition comprising the antibody construct of any one of claims 1 to 12.

14. The pharmaceutical composition of claim 13 for the use of treating and/or preventing an allergy.

15. The pharmaceutical composition of claim 13 for the use of preventing, reducing and/or alleviating allergic symptoms in a patient.


SEQUENCE LISTING

<110>  Medizinische Universität Wien

<120>  Antibody construct

<130>  46586

<160>  94

<170>  PatentIn version 3.5

<210>  1
<211>  114
<212>  PRT
<213>  Artificial Sequence

<220>
<223>  Antigen binding site or immunoglobulin or immunoglobulin fragment
       specific for Phl p 2

<400>  1

Leu Glu Ser Gly Pro Gly Leu Val Lys Pro Ala Gln Thr Leu Ser Leu
1               5                   10                  15

Ser Cys Ala Val Ser Gly Gly Ser Ile Arg Ser Gly Gly Tyr Tyr Trp
            20                  25                  30

Ser Trp Ile Arg Gln His Pro Gly Lys Gly Leu Glu Trp Ile Gly Tyr
        35                  40                  45

Ile Tyr His Ser Gly Asn Thr Tyr Tyr Asn Pro Ser Leu Lys Ser Arg
    50                  55                  60

Ile Ala Met Ser Val Asp Thr Ser Glu Asn Lys Phe Ser Leu Arg Leu
65                  70                  75                  80

Asn Ser Val Thr Ala Ala Asp Thr Ala Val Tyr Tyr Cys Ala Arg Leu
                85                  90                  95

Asp Gly Tyr Thr Leu Asp Ile Trp Gly Gln Gly Thr Leu Val Thr Val
            100                 105                 110

Ser Ser

<210>  2
<211>  106
<212>  PRT
<213>  Artificial Sequence

<220>
<223>  Antigen binding site or immunoglobulin or immunoglobulin fragment
       specific for Phl p 2

<400>  2

Glu Leu Thr Gln Ser Pro Ser Ser Leu Ser Ala Ser Val Gly Asp Arg
1               5                   10                  15

Val Thr Ile Ser Cys Arg Ala Ser Gln Arg Ile Asn Thr Tyr Leu Asn
            20                  25                  30

Trp Tyr Gln His Lys Pro Gly Lys Ala Pro Lys Leu Leu Ile Tyr Ala
        35                  40                  45

Ala Ser Ser Leu Gln Ser Gly Val Pro Ser Arg Phe Ser Gly Ser Gly
    50                  55                  60

Tyr Gly Thr Asp Phe Thr Leu Thr Ile Ser Ser Leu Gln Pro Glu Asp
65                  70                  75                  80

Phe Ala Ser Tyr Tyr Cys Gln Glu Ser Leu Ser Ala Ser Tyr Thr Phe
                85                  90                  95

Gly Gln Gly Thr Lys Val Glu Ile Lys Arg
            100                 105

<210>  3
<211>  259
<212>  PRT
<213>  Artificial Sequence

<220>
<223>  Antigen binding site or immunoglobulin or immunoglobulin fragment
       specific for Phl p 2

<400>  3

Met Ala Gln Val Gln Leu Gln Glu Ser Gly Pro Gly Leu Val Lys Pro
1               5                   10                  15

Ala Gln Thr Leu Ser Leu Ser Cys Ala Val Ser Gly Gly Ser Ile Arg
            20                  25                  30

Ser Gly Gly Tyr Tyr Trp Ser Trp Ile Arg Gln His Pro Gly Lys Gly
        35                  40                  45

Leu Glu Trp Ile Gly Tyr Ile Tyr His Ser Gly Asn Thr Tyr Tyr Asn
    50                  55                  60

Pro Ser Leu Lys Ser Arg Ile Ala Met Ser Val Asp Thr Ser Glu Asn
65                  70                  75                  80

Lys Phe Ser Leu Arg Leu Asn Ser Val Thr Ala Ala Asp Thr Ala Val

85                  90                  95

Tyr Tyr Cys Ala Arg Leu Asp Gly Tyr Thr Leu Asp Ile Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Glu Thr Thr Leu Thr Gln Ser Pro Ser
    130                 135                 140

Ser Leu Ser Ala Ser Val Gly Asp Arg Val Thr Ile Ser Cys Arg Ala
145                 150                 155                 160

Ser Gln Arg Ile Asn Thr Tyr Leu Asn Trp Tyr Gln His Lys Pro Gly
                165                 170                 175

Lys Ala Pro Lys Leu Leu Ile Tyr Ala Ala Ser Ser Leu Gln Ser Gly
            180                 185                 190

Val Pro Ser Arg Phe Ser Gly Ser Gly Tyr Gly Thr Asp Phe Thr Leu
        195                 200                 205

Thr Ile Ser Ser Leu Gln Pro Glu Asp Phe Ala Ser Tyr Tyr Cys Gln
    210                 215                 220

Glu Ser Leu Ser Ala Ser Tyr Thr Phe Gly Gln Gly Thr Lys Val Glu
225                 230                 235                 240

Ile Lys Arg Ala Ala Ala Gly Ala Pro Val Pro Tyr Pro Asp Pro Leu
                245                 250                 255

Glu Pro Arg

<210>  4
<211>  114
<212>  PRT
<213>  Artificial Sequence

<220>
<223>  Antigen binding site or immunoglobulin or immunoglobulin fragment
       specific for Phl p 2

<400>  4

Leu Glu Ser Gly Pro Gly Leu Val Lys Pro Ser Gln Thr Leu Ser Leu
1               5                   10                  15

Thr Cys Thr Val Ser Gly Gly Ser Ile Arg Ser Gly Gly Tyr Tyr Trp

20                  25                  30

Ser Trp Val Arg Gln Pro Pro Gly Lys Gly Leu Glu Trp Ile Gly Asn
        35                  40                  45

Ile Tyr His Ser Gly Asn Thr Tyr Tyr Asn Pro Ser Leu Lys Ser Arg
    50                  55                  60

Ile Thr Met Ser Val Asp Thr Ser Lys Asn His Phe Ser Leu Arg Leu
65                  70                  75                  80

Thr Ser Val Thr Ala Ala Asp Thr Ala Val Tyr Tyr Cys Ala Arg Ser
                85                  90                  95

Asp Gly Tyr Thr Leu Asp Asn Trp Gly Gln Gly Thr Leu Val Thr Val
            100                 105                 110

Ser Ser

<210>  5
<211>  106
<212>  PRT
<213>  Artificial Sequence

<220>
<223>  Antigen binding site or immunoglobulin or immunoglobulin fragment
       specific for Phl p 2

<400>  5

Glu Leu Thr Gln Ser Pro Ser Ser Leu Ser Ala Ser Val Gly Asp Arg
1               5                   10                  15

Val Thr Ile Thr Cys Arg Ala Arg Gln Ser Ile Ser Thr Tyr Leu Asn
            20                  25                  30

Trp Tyr Gln Gln Lys Pro Gly Lys Ala Pro Lys Leu Leu Ile Trp Ser
        35                  40                  45

Ala Ser Asn Leu Gln Ser Gly Val Pro Ser Arg Phe Ser Gly Ser Gly
    50                  55                  60

Ser Gly Thr Glu Phe Thr Leu Thr Ile Ser Asn Leu Gln Pro Glu Asp
65                  70                  75                  80

Phe Ala Ser Tyr Tyr Cys Gln Gln Ser Tyr Thr Thr Leu Tyr Thr Phe
                85                  90                  95

Gly Pro Gly Thr Lys Leu Glu Ile Lys Arg

            100                 105

<210> 6
<211> 114
<212> PRT
<213> Artificial Sequence

<220>
<223> Antigen binding site or immunoglobulin or immunoglobulin fragment
specific for Phl p 2

<400> 6

Leu Glu Ser Gly Pro Gly Leu Val Lys Pro Ser Gln Thr Leu Ser Leu
1               5                   10                  15

Thr Cys Thr Val Ser Gly Gly Ser Ile Arg Ser Gly Gly Tyr Tyr Trp
            20                  25                  30

Ser Trp Ile Arg Gln Pro Pro Gly Lys Gly Leu Glu Trp Ile Gly Tyr
        35                  40                  45

Ile Tyr His Ser Gly Asn Thr Tyr Tyr Asn Pro Ser Leu Lys Ser Arg
    50                  55                  60

Val Thr Met Ser Val Asp Thr Ser Lys Asn His Phe Ser Leu Arg Leu
65                  70                  75                  80

Ser Ser Val Thr Ala Ala Asp Thr Ala Val Tyr Tyr Cys Ala Arg Ser
                85                  90                  95

Asp Gly Tyr Thr Leu Asp Asn Trp Gly Gln Gly Thr Leu Val Thr Val
            100                 105                 110

Ser Ser

<210> 7
<211> 106
<212> PRT
<213> Artificial Sequence

<220>
<223> Antigen binding site or immunoglobulin or immunoglobulin fragment
specific for Phl p 2

<400> 7

Glu Leu Thr Gln Ser Pro Ser Ser Val Ser Ala Ser Val Gly Asp Arg
1               5                   10                  15

Val Thr Ile Thr Cys Arg Ala Ser Gln Gly Ile Ser Ser Trp Leu Ala
            20                  25                  30

Trp Tyr Gln Gln Lys Pro Gly Lys Ala Pro Lys Leu Leu Ile Tyr Ser
        35                  40                  45

Ala Ser Ser Leu Gln Ser Gly Val Pro Ser Arg Phe Ser Gly Ser Gly
    50                  55                  60

Ser Gly Thr Asp Phe Ser Leu Thr Ile Ser Ser Leu Gln Pro Glu Asp
65                  70                  75                  80

Ser Ala Thr Tyr Tyr Cys Gln Gln Ala Asn Ser Phe Pro Tyr Thr Phe
                85                  90                  95

Gly Gln Gly Thr Lys Val Glu Ile Lys Arg
            100                 105

<210>  8
<211>  10
<212>  PRT
<213>  Artificial Sequence

<220>
<223>  Antigen binding site or immunoglobulin or immunoglobulin fragment
       specific for Phl p 2

<400>  8

Gly Gly Ser Ile Arg Ser Gly Gly Tyr Tyr
1               5                   10

<210>  9
<211>  7
<212>  PRT
<213>  Artificial Sequence

<220>
<223>  Antigen binding site or immunoglobulin or immunoglobulin fragment
       specific for Phl p 2

<400>  9

Ile Tyr His Ser Gly Asn Thr
1               5

<210>  10
<211>  11
<212>  PRT
<213>  Artificial Sequence

<220>
<223>  Antigen binding site or immunoglobulin or immunoglobulin fragment
       specific for Phl p 2

<400>  10

Ala Arg Leu Asp Gly Tyr Thr Leu Asp Ile Trp
1               5                   10


<210>  11
<211>  6
<212>  PRT
<213>  Artificial Sequence

<220>
<223>  Antigen binding site or immunoglobulin or immunoglobulin fragment
       specific for Phl p 2

<400>  11

Gln Arg Ile Asn Thr Tyr
1               5


<210>  12
<211>  3
<212>  PRT
<213>  Artificial Sequence

<220>
<223>  Antigen binding site or immunoglobulin or immunoglobulin fragment
       specific for Phl p 2

<400>  12

Ala Ala Ser
1


<210>  13
<211>  9
<212>  PRT
<213>  Artificial Sequence

<220>
<223>  Antigen binding site or immunoglobulin or immunoglobulin fragment
       specific for Phl p 2

<400>  13

Gln Glu Ser Leu Ser Ala Ser Tyr Thr
1               5


<210>  14
<211>  10
<212>  PRT
<213>  Artificial Sequence

<220>
<223>  Antigen binding site or immunoglobulin or immunoglobulin fragment
       specific for Phl p 2

<400>  14

Ala Arg Ser Asp Gly Tyr Thr Leu Asp Asn
1               5                   10

<210>  15
<211>  6
<212>  PRT
<213>  Artificial Sequence

<220>
<223>  Antigen binding site or immunoglobulin or immunoglobulin fragment
       specific for Phl p 2

<400>  15

Gln Ser Ile Ser Thr Tyr
1               5


<210>  16
<211>  3
<212>  PRT
<213>  Artificial Sequence

<220>
<223>  Antigen binding site or immunoglobulin or immunoglobulin fragment
       specific for Phl p 2

<400>  16

Ser Ala Ser
1


<210>  17
<211>  9
<212>  PRT
<213>  Artificial Sequence

<220>
<223>  Antigen binding site or immunoglobulin or immunoglobulin fragment
       specific for Phl p 2

<400>  17

Gln Gln Ser Tyr Thr Thr Leu Tyr Thr
1               5


<210>  18
<211>  6
<212>  PRT
<213>  Artificial Sequence

<220>
<223>  Antigen binding site or immunoglobulin or immunoglobulin fragment
       specific for Phl p 2

<400>  18

Gln Gly Ile Ser Ser Tyr
1               5


<210>  19
<211>  9

<212>  PRT
<213>  Artificial Sequence

<220>
<223>  Antigen binding site or immunoglobulin or immunoglobulin fragment
       specific for Phl p 2

<400>  19

Gln Gln Ala Asn Ser Phe Pro Tyr Thr
1               5


<210>  20
<211>  117
<212>  PRT
<213>  Artificial Sequence

<220>
<223>  Antigen binding site or immunoglobulin or immunoglobulin fragment
       specific for Phl p 1

<400>  20

Leu Glu Ser Gly Gly Gly Trp Val Gln Pro Gly Arg Ser Leu Arg Leu
1               5                   10                  15


Ser Cys Ala Ala Ser Gly Phe Thr Phe Asp Asp His Ala Met His Trp
            20                  25                  30


Val Arg Gln Ala Pro Gly Lys Gly Leu Glu Trp Val Ala Gly Val Ser
        35                  40                  45


Trp Asn Ser Gly Thr Arg Ala Tyr Ala Asp Ser Val Lys Gly Arg Phe
    50                  55                  60


Thr Ile Ser Arg Asp Asn Ala Lys Asn Ser Leu Tyr Leu Glu Met Lys
65                  70                  75                  80


Ser Leu Arg Ala Glu Asp Thr Ala Ile Tyr Tyr Cys Ala Arg Asp Met
                85                  90                  95


Arg Ala Ala Thr Pro Tyr Ser Phe Asp Tyr Trp Gly Gln Gly Val Leu
            100                 105                 110


Val Thr Val Ser Ser
        115


<210>  21
<211>  108
<212>  PRT
<213>  Artificial Sequence

<220>
<223>  Antigen binding site or immunoglobulin or immunoglobulin fragment

specific for Phl p 1

<400> 21

Glu Leu Val Met Thr Gln Ser Pro Ser Ser Leu Ser Ala Ser Val Gly
1               5                   10                  15

Asp Arg Val Thr Ile Thr Cys Arg Ala Ser Gln Thr Phe Asn Asn Tyr
            20                  25                  30

Leu Asn Trp Tyr Gln Gln Lys Pro Gly Lys Ala Pro Asn Leu Leu Ile
        35                  40                  45

Tyr Ala Ala Ser Thr Leu Arg Arg Gly Ile Pro Ser Arg Phe Ser Gly
    50                  55                  60

Ser Gly Ser Gly Thr Tyr Phe Thr Leu Thr Ile Asn Gly Leu Gln Pro
65                  70                  75                  80

Glu Asp Phe Ala Thr Tyr Tyr Cys Gln Gln Ser Tyr Ser Thr Pro Leu
                85                  90                  95

Thr Phe Gly Gly Gly Thr Lys Val Asp Ile Lys Arg
            100                 105

<210> 22
<211> 117
<212> PRT
<213> Artificial Sequence

<220>
<223> Antigen binding site or immunoglobulin or immunoglobulin fragment
specific for Phl p 1

<400> 22

Leu Glu Ser Gly Gly Gly Leu Val Gln Pro Gly Gly Ser Leu Arg Leu
1               5                   10                  15

Ser Cys Ala Ala Ser Gly Phe Thr Phe Asp Asp His Ala Met His Trp
            20                  25                  30

Val Arg Gln Thr Pro Glu Lys Gly Leu Gln Trp Val Ser Gly Ile Ser
        35                  40                  45

Trp Asn Ser Gly Val Val Ala Tyr Ala Asp Ser Val Lys Gly Arg Phe
    50                  55                  60

Ser Ile Ser Arg Asp Asn Ala Lys Asn Ser Leu Tyr Leu Gln Met Asn
65                  70                  75                  80

Asn Leu Ile Pro Glu Asp Thr Ala Val Tyr Tyr Cys Val Arg Asp Phe
                85                  90                  95

Arg Ala Ala Ser Pro Tyr Phe Phe Asp Tyr Trp Gly Gln Gly Thr Arg
            100                 105                 110

Val Thr Val Ser Ser
        115

<210>  23
<211>  106
<212>  PRT
<213>  Artificial Sequence

<220>
<223>  Antigen binding site or immunoglobulin or immunoglobulin fragment
       specific for Phl p 1

<400>  23

Glu Leu Thr Gln Ser Pro Ser Ser Leu Ser Ala Ser Val Gly Asp Arg
1               5                   10                  15

Val Thr Ile Thr Cys Arg Ala Ser Gln Ser Ile Gly Asn Tyr Leu Asn
            20                  25                  30

Trp Tyr Gln Gln Lys Pro Gly Lys Ala Pro Asn Leu Leu Ile Tyr Ala
        35                  40                  45

Ala Ser Ser Leu Gln Ser Gly Val Pro Ser Arg Phe Ser Gly Ser Gly
    50                  55                  60

Ser Gly Thr Asp Phe Thr Leu Thr Ile Ser Ser Leu Gln Arg Glu Asp
65                  70                  75                  80

Phe Ala Thr Tyr Tyr Cys Gln Gln Ser Asn Arg Thr Pro Ile Thr Phe
                85                  90                  95

Gly Gln Gly Thr Arg Leu Glu Ile Lys Gly
            100                 105

<210>  24
<211>  262
<212>  PRT
<213>  Artificial Sequence

<220>
<223>  Antigen binding site or immunoglobulin or immunoglobulin fragment
       specific for Phl p 1

<400>  24

Met Ala Gln Val Asn Leu Arg Glu Ser Gly Gly Gly Leu Val Gln Pro

1               5                   10                  15

Gly Gly Ser Leu Arg Leu Ser Cys Ala Ala Ser Gly Phe Thr Phe Asp
            20                  25                  30

Asp His Ala Met His Trp Val Arg Gln Thr Pro Glu Lys Gly Leu Gln
        35                  40                  45

Trp Val Ser Gly Ile Ser Trp Asn Ser Gly Val Val Ala Tyr Ala Asp
    50                  55                  60

Ser Val Lys Gly Arg Phe Ser Ile Ser Arg Asp Asn Ala Lys Asn Ser
65                  70                  75                  80

Leu Tyr Leu Gln Met Asn Asn Leu Ile Pro Glu Asp Thr Ala Val Tyr
                85                  90                  95

Tyr Cys Val Arg Asp Phe Arg Ala Ala Thr Pro Tyr Phe Phe Asp Tyr
            100                 105                 110

Trp Gly Gln Gly Thr Leu Val Thr Val Ser Ser Gly Gly Gly Gly Ser
        115                 120                 125

Gly Gly Gly Gly Ser Gly Gly Gly Gly Ser Glu Thr Thr Leu Thr Gln
    130                 135                 140

Ser Pro Ser Ser Leu Ser Ala Ser Val Gly Asp Arg Val Thr Ile Thr
145                 150                 155                 160

Cys Arg Ala Ser Gln Ser Ile Gly Asn Tyr Leu Asn Trp Tyr Gln Gln
                165                 170                 175

Lys Pro Gly Lys Ala Pro Asn Leu Leu Ile Tyr Ala Ala Ser Ser Leu
            180                 185                 190

Gln Ser Gly Val Pro Ser Arg Phe Ser Gly Ser Gly Ser Gly Thr Asp
        195                 200                 205

Phe Thr Leu Thr Ile Ser Ser Leu Gln Arg Glu Asp Phe Ala Thr Tyr
    210                 215                 220

Tyr Cys Gln Gln Ser Asn Arg Thr Pro Ile Thr Phe Gly Gln Gly Thr
225                 230                 235                 240

Arg Leu Glu Ile Lys Arg Ala Ala Ala Gly Ala Pro Val Pro Tyr Pro
                245                 250                 255

Asp Pro Leu Glu Pro Arg
            260

<210>  25
<211>  117
<212>  PRT
<213>  Artificial Sequence

<220>
<223>  Antigen binding site or immunoglobulin or immunoglobulin fragment
       specific for Phl p 1

<400>  25

Leu Glu Ser Gly Gly Gly Trp Val Gln Pro Gly Arg Ser Leu Arg Leu
1               5                   10                  15

Ser Cys Ser Ala Ser Gly Leu Thr Phe Asp Asp Tyr Ala Met His Trp
            20                  25                  30

Val Arg Gln Ala Pro Gly Lys Gly Leu Glu Trp Val Ser Gly Ile Ser
        35                  40                  45

Trp Asn Ser Gly Val Arg Ala Tyr Ala Asp Ser Val Lys Gly Arg Phe
    50                  55                  60

Thr Ile Ser Arg Asp Asn Gly Lys Asn Ser Leu Tyr Leu Gln Met Asn
65                  70                  75                  80

Ser Leu Arg Pro Glu Asp Thr Ala Leu Tyr Tyr Cys Ala Lys Asp Ile
                85                  90                  95

Arg Ala Ala Thr Pro Tyr Ala Leu Asp His Trp Gly Gln Gly Val Leu
            100                 105                 110

Val Thr Val Ser Ser
        115

<210>  26
<211>  108
<212>  PRT
<213>  Artificial Sequence

<220>
<223>  Antigen binding site or immunoglobulin or immunoglobulin fragment
       specific for Phl p 1

<400>  26

Glu Leu Val Met Thr Gln Ser Pro Ser Ser Leu Ser Ala Ser Glu Gly
1               5                   10                  15

Asp Arg Val Thr Ile Thr Cys Arg Ala Ser Arg Thr Ile Tyr Asn Tyr

20                  25                  30

Leu Asn Trp Tyr Gln Gln Lys Pro Gly Arg Ala Pro Lys Leu Leu Ile
        35                  40                  45

His Ala Ala Ser Thr Leu Gln Asp Gly Val Pro Ser Arg Phe Ser Gly
    50                  55                  60

Ser Gly Ser Gly Thr Glu Phe Thr Leu Thr Ile Ser Gly Leu Gln Ser
65                  70                  75                  80

Glu Asp Phe Ala Thr Tyr Tyr Cys Gln Gln Ser His Gly Thr Pro Leu
                85                  90                  95

Thr Phe Gly Gly Gly Thr Thr Val Asp Val Lys Arg
            100                 105

<210>  27
<211>  259
<212>  PRT
<213>  Artificial Sequence

<220>
<223>  Antigen binding site or immunoglobulin or immunoglobulin fragment
       specific for Phl p 1

<400>  27

Met Ala Gln Val Gln Leu Gln Glu Ser Gly Gly Gly Leu Val Gln Pro
1               5                   10                  15

Gly Gly Ser Leu Lys Leu Ser Cys Ala Ala Ser Glu Phe Thr Phe Asn
            20                  25                  30

Asn Tyr Trp Met Ser Trp Val Arg Gln Ala Pro Gly Lys Gly Leu Glu
        35                  40                  45

Trp Val Ala Asn Ile Lys Pro Asp Gly Ser Val Lys Tyr Tyr Val Asp
    50                  55                  60

Ser Val Lys Gly Arg Phe Thr Ile Ser Arg Asp Asn Ala Lys Asn Ser
65                  70                  75                  80

Leu Phe Leu Gln Met Asn Ser Leu Arg Ala Glu Asp Thr Ala Val Tyr
                85                  90                  95

Phe Cys Ala Arg Glu Arg Gln Ser Gly Ala Phe Gly Leu Trp Gly Gln
            100                 105                 110

Gly Thr Thr Val Thr Val Ser Ser Gly Ala Gly Gly Ser Gly Gly Gly

115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Ser Ile Ser Ser Tyr Leu Asn Trp Tyr Gln Gln Lys Pro Gly
                165                 170                 175

Lys Ala Pro Lys Leu Leu Ile Tyr Ala Ala Ser Ser Leu Gln Ser Gly
            180                 185                 190

Val Pro Ser Arg Phe Ser Gly Ser Gly Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln Ser Tyr Ser Thr Pro Leu Thr Phe Gly Gly Gly Thr Lys Val Glu
225                 230                 235                 240

Ile Lys Arg Ala Ala Ala Gly Ala Pro Val Pro Tyr Pro Asp Pro Leu
                245                 250                 255

Glu Pro Arg

<210>  28
<211>  269
<212>  PRT
<213>  Artificial Sequence

<220>
<223>  Antigen binding site or immunoglobulin or immunoglobulin fragment
       specific for Phl p 1

<400>  28

Met Ala Gln Val Asn Leu Arg Glu Ser Gly Gly Gly Leu Val Arg Pro
1               5                   10                  15

Gly Arg Ser Leu Arg Leu Ser Cys Ala Ala Ser Gly Phe Thr Phe Asp
            20                  25                  30

Asp Tyr Ala Met His Trp Val Arg Gln Ala Pro Gly Lys Gly Leu Glu
        35                  40                  45

Trp Val Ser Gly Ile Ser Trp Asn Ser Gly Ser Ile Gly Tyr Ala Asp

50                  55                  60

Ser Val Lys Gly Arg Phe Thr Ile Ser Arg Asp Asn Ala Lys Asn Ser
65                  70                  75                  80

Leu Tyr Leu Gln Met Asn Ser Leu Arg Ala Glu Asp Thr Ala Val Tyr
                85                  90                  95

Tyr Cys Ala Arg Glu Trp Asp Gly Thr Ala Tyr Tyr Gly Tyr Thr Val
            100                 105                 110

Gly Tyr Trp Gly Gln Gly Thr Leu Val Thr Val Ser Ser Gly Gly Gly
        115                 120                 125

Gly Ser Gly Gly Gly Gly Ser Gly Gly Gly Gly Ser Asp Val Val Met
    130                 135                 140

Thr Gln Ser Pro Leu Ser Leu Pro Val Thr Leu Gly Gln Pro Ala Ser
145                 150                 155                 160

Ile Ser Cys Arg Ser Ser Gln Ser Leu Val Tyr Ser Asp Gly Asn Thr
                165                 170                 175

Tyr Leu Asn Trp Phe Gln Gln Arg Pro Gly Gln Ser Pro Arg Arg Leu
            180                 185                 190

Ile Tyr Lys Val Ser Asn Arg Asp Ser Gly Val Pro Asp Arg Phe Ser
        195                 200                 205

Gly Ser Gly Ser Gly Thr Asp Phe Thr Leu Lys Ile Ser Arg Val Glu
    210                 215                 220

Ala Glu Asp Val Gly Val Tyr Tyr Cys Met Gln Gly Thr His Trp Pro
225                 230                 235                 240

Pro Thr Phe Gly Gln Gly Thr Arg Leu Glu Ile Lys Arg Ala Ala Ala
                245                 250                 255

Gly Ala Pro Val Pro Tyr Pro Asp Pro Leu Glu Pro Arg
            260                 265

<210>  29
<211>  271
<212>  PRT
<213>  Artificial Sequence

<220>
<223>  Antigen binding site or immunoglobulin or immunoglobulin fragment
       specific for Phl p 1

<400>  29

Met Ala Gln Val Gln Leu Gln Glu Ser Gly Gly Gly Val Val Gln Pro
1               5                   10                  15

Gly Arg Ser Leu Arg Leu Ser Cys Ala Ala Ser Gly Phe Thr Phe Ser
            20                  25                  30

Ser Tyr Ala Met Ser Trp Val Arg Gln Ala Pro Gly Lys Gly Leu Glu
        35                  40                  45

Trp Val Ser Gly Ile Ser Gly Ser Gly Gly Ser Thr Tyr Tyr Ala Asp
    50                  55                  60

Ser Val Lys Gly Arg Phe Thr Ile Ser Arg Asp Asn Ser Lys Asn Thr
65                  70                  75                  80

Leu Tyr Leu Gln Met Asn Ser Leu Arg Ala Glu Asp Thr Ala Ile Tyr
                85                  90                  95

Tyr Cys Ala Arg Val Leu Arg Asn Gln Ala Ala Ala Gly Thr Ile Arg
            100                 105                 110

Trp Phe Asp Pro Trp Gly Gln Gly Thr Leu Val Thr Val Ser Ser Gly
        115                 120                 125

Gly Gly Gly Ser Gly Gly Gly Gly Ser Gly Gly Gly Gly Ser Asp Val
    130                 135                 140

Val Met Thr Gln Ser Pro Leu Ser Leu Pro Val Thr Leu Gly Gln Pro
145                 150                 155                 160

Ala Ser Ile Ser Cys Arg Ser Ser Gln Ser Leu Val Tyr Ser Asp Gly
                165                 170                 175

Asn Thr Tyr Leu Asn Trp Phe Gln Gln Arg Pro Gly Gln Ser Pro Arg
            180                 185                 190

Arg Leu Ile Tyr Lys Val Ser Asn Arg Asp Ser Gly Val Pro Asp Arg
        195                 200                 205

Phe Ser Gly Ser Gly Ser Gly Thr Asp Phe Thr Leu Lys Ile Arg Arg
    210                 215                 220

Val Glu Ala Glu Asp Val Gly Val Tyr Tyr Cys Met Gln Gly Thr His
225                 230                 235                 240

Trp Pro Pro Thr Phe Gly Gln Gly Thr Lys Leu Glu Ile Lys Arg Ala
                245                 250                 255

Ala Ala Gly Ala Pro Val Pro Tyr Pro Asp Pro Leu Glu Pro Arg
            260                 265                 270

<210>  30
<211>  8
<212>  PRT
<213>  Artificial Sequence

<220>
<223>  Antigen binding site or immunoglobulin or immunoglobulin fragment
       specific for Phl p 1

<400>  30

Gly Phe Thr Phe Asp Asp His Ala
1               5

<210>  31
<211>  8
<212>  PRT
<213>  Artificial Sequence

<220>
<223>  Antigen binding site or immunoglobulin or immunoglobulin fragment
       specific for Phl p 1

<400>  31

Val Ser Trp Asn Ser Gly Thr Arg
1               5

<210>  32
<211>  14
<212>  PRT
<213>  Artificial Sequence

<220>
<223>  Antigen binding site or immunoglobulin or immunoglobulin fragment
       specific for Phl p 1

<400>  32

Ala Arg Asp Met Arg Ala Ala Thr Pro Tyr Ser Phe Asp Tyr
1               5                   10

<210>  33
<211>  6
<212>  PRT
<213>  Artificial Sequence

<220>
<223>  Antigen binding site or immunoglobulin or immunoglobulin fragment
       specific for Phl p 1

<400>  33

Gln Thr Phe Asn Asn Tyr
1               5


<210>  34
<211>  9
<212>  PRT
<213>  Artificial Sequence

<220>
<223>  Antigen binding site or immunoglobulin or immunoglobulin fragment
       specific for Phl p 1

<400>  34

Gln Gln Ser Tyr Ser Thr Pro Leu Thr
1               5


<210>  35
<211>  8
<212>  PRT
<213>  Artificial Sequence

<220>
<223>  Antigen binding site or immunoglobulin or immunoglobulin fragment
       specific for Phl p 1

<400>  35

Ile Ser Trp Asn Ser Gly Val Val
1               5


<210>  36
<211>  14
<212>  PRT
<213>  Artificial Sequence

<220>
<223>  Antigen binding site or immunoglobulin or immunoglobulin fragment
       specific for Phl p 1

<400>  36

Val Arg Asp Phe Arg Ala Ala Ser Pro Tyr Phe Phe Asp Tyr
1               5                   10


<210>  37
<211>  6
<212>  PRT
<213>  Artificial Sequence

<220>
<223>  Antigen binding site or immunoglobulin or immunoglobulin fragment
       specific for Phl p 1

<400>  37

Gln Ser Ile Gly Asn Tyr
1               5

<210>  38
<211>  9
<212>  PRT
<213>  Artificial Sequence

<220>
<223>  Antigen binding site or immunoglobulin or immunoglobulin fragment
       specific for Phl p 1

<400>  38

Gln Gln Ser Asn Arg Thr Pro Ile Thr
1               5


<210>  39
<211>  8
<212>  PRT
<213>  Artificial Sequence

<220>
<223>  Antigen binding site or immunoglobulin or immunoglobulin fragment
       specific for Phl p 1

<400>  39

Gly Leu Thr Phe Asp Asp Tyr Ala
1               5


<210>  40
<211>  8
<212>  PRT
<213>  Artificial Sequence

<220>
<223>  Antigen binding site or immunoglobulin or immunoglobulin fragment
       specific for Phl p 1

<400>  40

Ile Ser Trp Asn Ser Gly Val Arg
1               5


<210>  41
<211>  14
<212>  PRT
<213>  Artificial Sequence

<220>
<223>  Antigen binding site or immunoglobulin or immunoglobulin fragment
       specific for Phl p 1

<400>  41

Ala Lys Asp Ile Arg Ala Ala Thr Pro Tyr Ala Leu Asp His
1               5                   10


<210>  42

<211>  6
<212>  PRT
<213>  Artificial Sequence

<220>
<223>  Antigen binding site or immunoglobulin or immunoglobulin fragment
       specific for Phl p 1

<400>  42

Arg Thr Ile Tyr Asn Tyr
1               5


<210>  43
<211>  9
<212>  PRT
<213>  Artificial Sequence

<220>
<223>  Antigen binding site or immunoglobulin or immunoglobulin fragment
       specific for Phl p 1

<400>  43

Gln Gln Ser His Gly Thr Pro Leu Thr
1               5


<210>  44
<211>  8
<212>  PRT
<213>  Artificial Sequence

<220>
<223>  Antigen binding site or immunoglobulin or immunoglobulin fragment
       specific for Phl p 1

<400>  44

Glu Phe Thr Phe Asn Asn Tyr Trp
1               5


<210>  45
<211>  8
<212>  PRT
<213>  Artificial Sequence

<220>
<223>  Antigen binding site or immunoglobulin or immunoglobulin fragment
       specific for Phl p 1

<400>  45

Ile Lys Pro Asp Gly Ser Val Lys
1               5


<210>  46
<211>  11
<212>  PRT
<213>  Artificial Sequence

<220>
<223>  Antigen binding site or immunoglobulin or immunoglobulin fragment
      specific for Phl p 1

<400>  46

Ala Arg Glu Arg Gln Ser Gly Ala Phe Gly Leu
1               5                   10


<210>  47
<211>  6
<212>  PRT
<213>  Artificial Sequence

<220>
<223>  Antigen binding site or immunoglobulin or immunoglobulin fragment
      specific for Phl p 1

<400>  47

Gln Ser Ile Ser Ser Tyr
1               5


<210>  48
<211>  8
<212>  PRT
<213>  Artificial Sequence

<220>
<223>  Antigen binding site or immunoglobulin or immunoglobulin fragment
      specific for Phl p 1

<400>  48

Gly Phe Thr Phe Asp Asp Tyr Ala
1               5


<210>  49
<211>  8
<212>  PRT
<213>  Artificial Sequence

<220>
<223>  Antigen binding site or immunoglobulin or immunoglobulin fragment
      specific for Phl p 1

<400>  49

Ile Ser Trp Asn Ala Gly Ser Ile
1               5


<210>  50
<211>  16
<212>  PRT
<213>  Artificial Sequence

<220>
<223>  Antigen binding site or immunoglobulin or immunoglobulin fragment

specific for Phl p 1

<400> 50

Ala Arg Glu Trp Asp Gly Thr Ala Tyr Tyr Gly Tyr Thr Val Gly Tyr
1               5                   10                  15


<210> 51
<211> 11
<212> PRT
<213> Artificial Sequence

<220>
<223> Antigen binding site or immunoglobulin or immunoglobulin fragment
specific for Phl p 1

<400> 51

Gln Ser Leu Val Tyr Ser Asp Gly Asn Thr Tyr
1               5                   10


<210> 52
<211> 3
<212> PRT
<213> Artificial Sequence

<220>
<223> Antigen binding site or immunoglobulin or immunoglobulin fragment
specific for Phl p 1

<400> 52

Lys Val Ser
1


<210> 53
<211> 9
<212> PRT
<213> Artificial Sequence

<220>
<223> Antigen binding site or immunoglobulin or immunoglobulin fragment
specific for Phl p 1

<400> 53

Met Gln Gly Thr His Trp Pro Pro Thr
1               5


<210> 54
<211> 8
<212> PRT
<213> Artificial Sequence

<220>
<223> Antigen binding site or immunoglobulin or immunoglobulin fragment
specific for Phl p 1

<400> 54

Gly Phe Thr Phe Ser Ser Tyr Ala
1               5


<210>  55
<211>  8
<212>  PRT
<213>  Artificial Sequence

<220>
<223>  Antigen binding site or immunoglobulin or immunoglobulin fragment
       specific for Phl p 1

<400>  55

Ile Ser Gly Ser Gly Gly Ser Thr
1               5


<210>  56
<211>  18
<212>  PRT
<213>  Artificial Sequence

<220>
<223>  Antigen binding site or immunoglobulin or immunoglobulin fragment
       specific for Phl p 1

<400>  56

Ala Arg Val Leu Arg Asn Gln Ala Ala Ala Gly Thr Ile Arg Trp Glu
1               5                   10                  15


Asp Pro


<210>  57
<211>  10
<212>  PRT
<213>  Artificial Sequence

<220>
<223>  Antigen binding site or immunoglobulin or immunoglobulin fragment
       specific for Phl p 1

<400>  57

Gln Ser Leu Val Tyr Ser Asp Gly Asn Thr
1               5                   10


<210>  58
<211>  276
<212>  PRT
<213>  Artificial Sequence

<220>
<223>  Antigen binding site or immunoglobulin or immunoglobulin fragment
       specific for Bet v 1

<400>  58

Met Ala Gln Val Gln Leu Val Gln Ser Gly Ala Glu Val Lys Lys Pro
1               5                   10                  15

Gly Ser Ser Val Lys Val Ser Cys Lys Ala Ser Gly Gly Thr Phe Ser
            20                  25                  30

Ser Tyr Ala Ile Ser Trp Val Arg Gln Ala Pro Gly Gln Gly Leu Glu
        35                  40                  45

Trp Met Gly Arg Ile Ile Pro Ile Leu Gly Ile Val Asn Tyr Ala Gln
    50                  55                  60

Lys Phe Gln Gly Arg Val Thr Ile Thr Ala Asp Lys Ser Thr Ser Thr
65                  70                  75                  80

Ala Tyr Met Glu Leu Ser Ser Leu Arg Ser Glu Asp Thr Ala Val Tyr
                85                  90                  95

Tyr Cys Ala Arg Glu Pro Leu Gly Tyr Cys Ser Gly Gly Ser Cys Tyr
            100                 105                 110

Pro Leu His Asn Gly Met Asp Val Trp Gly Gln Gly Thr Thr Val Thr
        115                 120                 125

Val Ser Ser Ser Gly Gly Gly Ser Gly Gly Gly Gly Ser Gly Gly Gly
    130                 135                 140

Gly Ser Asp Ile Gln Met Thr Gln Ser Pro Asp Ser Leu Ala Val Ser
145                 150                 155                 160

Leu Gly Glu Arg Ala Thr Ile Asn Cys Lys Ser Ser Gln Ser Val Leu
                165                 170                 175

Tyr Ser Ser Asn Asn Lys Asn Tyr Leu Ala Trp Tyr Gln Gln Lys Pro
            180                 185                 190

Gly Gln Pro Pro Lys Leu Leu Phe Tyr Trp Ala Ser Thr Arg Glu Ser
        195                 200                 205

Gly Val Pro Asp Arg Phe Ser Gly Ser Gly Ser Gly Thr Asp Phe Thr
    210                 215                 220

Leu Thr Ile Ser Ser Leu Gln Ala Glu Asp Val Ala Val Tyr Tyr Cys
225                 230                 235                 240

Gln Gln Tyr Tyr Thr Thr Pro Leu Thr Phe Gly Gln Gly Thr Lys Leu

245                 250                 255

Glu Ile Lys Arg Ala Ala Ala Gly Ala Pro Val Pro Tyr Pro Asp Pro
            260                 265                 270

Leu Glu Pro Arg
        275

<210>  59
<211>  8
<212>  PRT
<213>  Artificial Sequence

<220>
<223>  Antigen binding site or immunoglobulin or immunoglobulin fragment
       specific for Bet v 1

<400>  59

Gly Gly Thr Phe Ser Ser Tyr Ala
1               5

<210>  60
<211>  8
<212>  PRT
<213>  Artificial Sequence

<220>
<223>  Antigen binding site or immunoglobulin or immunoglobulin fragment
       specific for Bet v 1

<400>  60

Ile Ile Pro Ile Leu Gly Ile Val
1               5

<210>  61
<211>  22
<212>  PRT
<213>  Artificial Sequence

<220>
<223>  Antigen binding site or immunoglobulin or immunoglobulin fragment
       specific for Bet v 1

<400>  61

Ala Arg Glu Pro Leu Gly Tyr Cys Ser Gly Gly Ser Cys Tyr Pro Leu
1               5                   10                  15

His Asn Gly Met Asp Val
            20

<210>  62
<211>  12
<212>  PRT

<213>  Artificial Sequence

<220>
<223>  Antigen binding site or immunoglobulin or immunoglobulin fragment
       specific for Bet v 1

<400>  62

Gln Ser Val Leu Tyr Ser Ser Asn Asn Lys Asn Tyr
1               5                   10


<210>  63
<211>  3
<212>  PRT
<213>  Artificial Sequence

<220>
<223>  Antigen binding site or immunoglobulin or immunoglobulin fragment
       specific for Bet v 1

<400>  63

Trp Ala Ser
1


<210>  64
<211>  9
<212>  PRT
<213>  Artificial Sequence

<220>
<223>  Antigen binding site or immunoglobulin or immunoglobulin fragment
       specific for Bet v 1

<400>  64

Gln Gln Tyr Tyr Thr Thr Pro Leu Thr
1               5


<210>  65
<211>  132
<212>  PRT
<213>  Artificial Sequence

<220>
<223>  Antigen binding site or immunoglobulin or immunoglobulin fragment
       specific for Bet v 1

<400>  65

Met Glu Phe Gly Leu Ser Trp Leu Phe Leu Val Ala Phe Leu Lys Gly
1               5                   10                  15


Val Gln Cys Glu Val Gln Leu Leu Glu Ser Gly Gly Gly Leu Val Gln
            20                  25                  30


Pro Gly Gly Ser Leu Arg Leu Ser Cys Val Ala Ser Gly Phe Thr Phe
        35                  40                  45

Thr Asn Tyr Ala Met Thr Trp Val Arg Gln Ala Pro Gly Lys Gly Leu
    50                  55                  60

Glu Trp Val Ser Ala Ile Ser Asn Tyr Gly Gly Ser Thr Tyr Tyr Ser
65                  70                  75                  80

Asp Ser Val Lys Gly Arg Phe Thr Ile Ser Arg Asp Asn Ser Lys Ser
                85                  90                  95

Thr Leu Ser Leu His Met Asn Ser Leu Arg Ala Glu Asp Thr Ala Leu
            100                 105                 110

Tyr Tyr Cys Ala Lys Tyr Phe Asp Phe Trp Gly Gln Gly Thr Leu Val
        115                 120                 125

Thr Val Ser Ser
    130

<210>  66
<211>  128
<212>  PRT
<213>  Artificial Sequence

<220>
<223>  Antigen binding site or immunoglobulin or immunoglobulin fragment
       specific for Bet v 1

<400>  66

Met Arg Leu Pro Ala Gln Leu Leu Gly Leu Leu Leu Leu Trp Val Pro
1               5                   10                  15

Gly Ala Gly Cys Asp Ile Gln Leu Thr Gln Ser Pro Ser Ser Leu Ser
            20                  25                  30

Ala Ser Val Gly Asp Arg Val Thr Ile Thr Cys Arg Val Ser Gln Gly
        35                  40                  45

Ile Ser Asn Cys Leu Asn Trp Tyr Arg Arg Lys Pro Gly Lys Val Asn
    50                  55                  60

Lys Val Leu Phe Tyr Ser Ala Ser Asn Phe Gln Ser Gly Val Pro Ser
65                  70                  75                  80

Arg Phe Ser Ala Ser Ala Ser Gly Thr Asp Leu Thr Leu Ser Val Asn
                85                  90                  95

Thr Leu Gln Pro Glu Asp Val Ala Thr Cys Tyr Cys Gln Arg Thr Asp
            100                 105                 110

Asn Ala Leu His His Phe Ala Gln Gly Thr Arg Leu Glu Ile Lys Arg
        115                 120                 125

<210>  67
<211>  8
<212>  PRT
<213>  Artificial Sequence

<220>
<223>  Antigen binding site or immunoglobulin or immunoglobulin fragment
       specific for Bet v 1

<400>  67

Gly Phe Thr Phe Thr Asn Tyr Ala
1               5

<210>  68
<211>  8
<212>  PRT
<213>  Artificial Sequence

<220>
<223>  Antigen binding site or immunoglobulin or immunoglobulin fragment
       specific for Bet v 1

<400>  68

Ile Ser Asn Tyr Gly Gly Ser Thr
1               5

<210>  69
<211>  6
<212>  PRT
<213>  Artificial Sequence

<220>
<223>  Antigen binding site or immunoglobulin or immunoglobulin fragment
       specific for Bet v 1

<400>  69

Ala Lys Tyr Phe Asp Phe
1               5

<210>  70
<211>  6
<212>  PRT
<213>  Artificial Sequence

<220>
<223>  Antigen binding site or immunoglobulin or immunoglobulin fragment
       specific for Bet v 1

<400>  70

Gln Gly Ile Ser Asn Cys

1               5


<210>  71
<211>  9
<212>  PRT
<213>  Artificial Sequence

<220>
<223>  Antigen binding site or immunoglobulin or immunoglobulin fragment
       specific for Bet v 1

<400>  71

Gln Arg Thr Asp Asn Ala Leu His His
1               5


<210>  72
<211>  120
<212>  PRT
<213>  Artificial Sequence

<220>
<223>  Antigen binding site or immunoglobulin or immunoglobulin fragment
       specific for Phl p 5

<400>  72

Leu Glu Ser Gly Gly Gly Val Val Gln Pro Gly Arg Ser Leu Arg Leu
1               5                   10                  15


Ser Cys Ala Ala Ser Glu Phe Ala Phe Asp Ser Tyr Glu Ile Tyr Trp
            20                  25                  30


Val Arg Gln Ala Pro Gly Lys Gly Leu Glu Trp Val Ala Met Ile Ser
        35                  40                  45


Ile Asp Glu Thr Asn Lys His Tyr Ala Asp Ser Val Lys Gly Arg Phe
    50                  55                  60


Thr Ile Phe Arg Asp Asn Ser Asn Lys Thr Val His Leu Gln Met Asn
65                  70                  75                  80


Asn Leu Arg Ser Glu Asp Ser Gly Val Tyr Tyr Cys Ala Arg Trp Asn
                85                  90                  95


Asn Trp Asn His Arg Gly Met Val Phe Ala Phe Asp Leu Trp Gly Gln
            100                 105                 110


Gly Thr Met Val Thr Val Ser Ser
        115                 120


<210>  73
<211>  106

<212>  PRT
<213>  Artificial Sequence

<220>
<223>  Antigen binding site or immunoglobulin or immunoglobulin fragment
       specific for Phl p 5

<400>  73

Glu Leu Thr Gln Ser Pro Ser Ser Val Ser Ala Ser Val Gly Asp Arg
1               5                   10                  15

Val Thr Ile Thr Cys Arg Ala Ser Gln Gly Ile Ser Ser Trp Leu Ala
            20                  25                  30

Trp Tyr Gln Gln Lys Pro Gly Lys Ala Pro Lys Leu Leu Ile Tyr Ser
        35                  40                  45

Ala Ser Ser Leu Gln Ser Gly Val Pro Ser Arg Phe Ser Gly Ser Gly
    50                  55                  60

Ser Gly Thr Asp Phe Ser Leu Thr Ile Ser Ser Leu Gln Pro Glu Asp
65                  70                  75                  80

Ser Ala Thr Tyr Tyr Cys Gln Gln Ala Asn Ser Phe Pro Tyr Thr Phe
                85                  90                  95

Gly Gln Gly Thr Lys Val Glu Ile Lys Arg
            100                 105


<210>  74
<211>  108
<212>  PRT
<213>  Artificial Sequence

<220>
<223>  Antigen binding site or immunoglobulin or immunoglobulin fragment
       specific for Phl p 5

<400>  74

Glu Leu Val Met Thr Gln Ser Pro Ser Ser Val Ser Ala Ser Val Gly
1               5                   10                  15

Asp Arg Val Thr Ile Thr Cys Arg Ala Ser His Ser Ile Ser Asn Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asn Tyr Tyr Cys Gln Glu Ser Phe Ser Pro Ser Gly
                85                  90                  95

Thr Phe Gly Gln Gly Thr Lys Val Glu Ile Lys Arg
            100                 105

<210>  75
<211>  106
<212>  PRT
<213>  Artificial Sequence

<220>
<223>  Antigen binding site or immunoglobulin or immunoglobulin fragment
       specific for Phl p 5

<400>  75

Glu Leu Thr Gln Ser Pro Ser Ser Leu Ser Ala Ser Val Gly Asp Arg
1               5                   10                  15

Val Thr Ile Thr Cys Arg Ala Ser Gln Ser Ile Leu Gly Tyr Leu Asn
            20                  25                  30

Trp Tyr Gln Gln Lys Pro Gly Lys Ala Pro Lys Leu Leu Ile Tyr Ala
        35                  40                  45

Ala Ser Thr Leu Gln Ser Gly Val Pro Ser Arg Phe Ser Gly Ser Gly
    50                  55                  60

Ser Gly Thr Asp Phe Thr Leu Thr Ile Thr Ser Leu Gln Pro Asp Asp
65                  70                  75                  80

Phe Ala Thr Tyr Tyr Cys Gln Gln Ser Tyr Ile Thr Pro Arg Thr Phe
                85                  90                  95

Gly Gln Gly Thr Lys Val Glu Val Lys Arg
            100                 105

<210>  76
<211>  108
<212>  PRT
<213>  Artificial Sequence

<220>
<223>  Antigen binding site or immunoglobulin or immunoglobulin fragment
       specific for Phl p 5

<400>  76

Glu Leu Val Met Thr Gln Ser Pro Ser Ser Leu Ser Ala Phe Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser His Ser Thr Pro Tyr
                85                  90                  95

Thr Phe Gly Gln Gly Thr Asn Leu Glu Ile Lys Arg
            100                 105

<210>  77
<211>  8
<212>  PRT
<213>  Artificial Sequence

<220>
<223>  Antigen binding site or immunoglobulin or immunoglobulin fragment
       specific for Phl p 5

<400>  77

Glu Phe Ala Phe Asp Ser Tyr Glu
1               5

<210>  78
<211>  8
<212>  PRT
<213>  Artificial Sequence

<220>
<223>  Antigen binding site or immunoglobulin or immunoglobulin fragment
       specific for Phl p 5

<400>  78

Ile Ser Ile Asp Glu Thr Asn Lys
1               5

<210>  79
<211>  17
<212>  PRT
<213>  Artificial Sequence

<220>
<223>  Antigen binding site or immunoglobulin or immunoglobulin fragment
      specific for Phl p 5

<400>  79

Ala Arg Trp Asn Asn Trp Asn His Arg Gly Met Val Phe Ala Phe Asp
1               5                   10                  15

Leu

<210>  80
<211>  6
<212>  PRT
<213>  Artificial Sequence

<220>
<223>  Antigen binding site or immunoglobulin or immunoglobulin fragment
      specific for Phl p 5

<400>  80

Gln Gly Ile Ser Ser Trp
1               5

<210>  81
<211>  6
<212>  PRT
<213>  Artificial Sequence

<220>
<223>  Antigen binding site or immunoglobulin or immunoglobulin fragment
      specific for Phl p 5

<400>  81

His Ser Ile Ser Asn Tyr
1               5

<210>  82
<211>  9
<212>  PRT
<213>  Artificial Sequence

<220>
<223>  Antigen binding site or immunoglobulin or immunoglobulin fragment
      specific for Phl p 5

<400>  82

Gln Glu Ser Phe Ser Pro Ser Gly Thr
1               5

<210>  83
<211>  6
<212>  PRT
<213>  Artificial Sequence

<220>
<223>  Antigen binding site or immunoglobulin or immunoglobulin fragment
       specific for Phl p 5

<400>  83

Gln Ser Ile Leu Gly Tyr
1               5


<210>  84
<211>  9
<212>  PRT
<213>  Artificial Sequence

<220>
<223>  Antigen binding site or immunoglobulin or immunoglobulin fragment
       specific for Phl p 5

<400>  84

Gln Gln Ser Tyr Ile Thr Pro Arg Thr
1               5


<210>  85
<211>  9
<212>  PRT
<213>  Artificial Sequence

<220>
<223>  Antigen binding site or immunoglobulin or immunoglobulin fragment
       specific for Phl p 5

<400>  85

Gln Gln Ser His Ser Thr Pro Tyr Thr
1               5


<210>  86
<211>  256
<212>  PRT
<213>  Artificial Sequence

<220>
<223>  Antigen binding site or immunoglobulin or immunoglobulin fragment
       specific for Phl p 6

<400>  86

Glu Val Gln Leu Val Gln Ser Gly Gly Gly Leu Val Lys Pro Gly Gly
1               5                   10                  15


Ser Leu Arg Leu Ser Cys Ala Ala Ser Gly Phe Ile Phe Ser Asp His
            20                  25                  30


Tyr Met Ala Trp Val Arg Gln Thr Pro Gly Lys Gly Leu Glu Trp Ile
        35                  40                  45

Ser Tyr Ile Ser Met Thr Ser Ser Tyr Thr Asn Tyr Ala Asp Ser Val
    50                  55                  60

Lys Gly Arg Phe Thr Ile Ser Arg Asp Asn Ala Lys Asn Ser Leu Ser
65                  70                  75                  80

Leu Glu Met Asn Ser Leu Arg Val Glu Asp Thr Ala Val Tyr Tyr Cys
                85                  90                  95

Ala Arg Asp Arg Leu Arg Asn Trp Asn Asp Asp Ala Phe Asp Ile Trp
            100                 105                 110

Gly Pro Gly Thr Met Val Thr Val Ser Ser Gly Gly Gly Gly Ser Gly
        115                 120                 125

Gly Gly Gly Ser Gly Gly Gly Gly Ser Ser Tyr Glu Leu Thr Gln Pro
    130                 135                 140

Ala Ser Val Ser Gly Ser Pro Gly Gln Ser Ile Thr Ile Ser Cys Thr
145                 150                 155                 160

Gly Thr Pro Ser Asp Ile Gly Thr Tyr Pro Tyr Val Ser Trp Tyr Gln
                165                 170                 175

Gln His Pro Asp Arg Ala Pro Lys Leu Leu Ile Tyr Gly Val Thr Asn
            180                 185                 190

Arg Pro Ser Gly Val Ser Asn Arg Phe Ser Gly Ser Lys Ser Gly Asn
        195                 200                 205

Thr Ala Ser Leu Thr Ile Ser Glu Leu Gln Ala Glu Asp Glu Ala Asp
    210                 215                 220

Tyr Tyr Cys Ser Ser Tyr Thr Leu Thr Ser Ile Val Val Phe Gly Gly
225                 230                 235                 240

Gly Thr Lys Leu Thr Val Leu Gly Asp Tyr Lys Asp Asp Asp Asp Lys
                245                 250                 255

<210>  87
<211>  122
<212>  PRT
<213>  Artificial Sequence

<220>
<223>  Antigen binding site or immunoglobulin or immunoglobulin fragment
       specific for Phl p 6

<400>  87

Glu Val Gln Leu Val Gln Ser Gly Gly Gly Leu Val Lys Pro Gly Gly
1               5                   10                  15

Ser Leu Arg Leu Ser Cys Ala Ala Ser Gly Phe Ile Phe Ser Asp His
            20                  25                  30

Tyr Met Ala Trp Val Arg Gln Thr Pro Gly Lys Gly Leu Glu Trp Ile
        35                  40                  45

Ser Tyr Ile Ser Met Thr Ser Ser Tyr Thr Asn Tyr Ala Asp Ser Val
    50                  55                  60

Lys Gly Arg Phe Thr Ile Ser Arg Asp Asn Ala Lys Asn Ser Leu Ser
65                  70                  75                  80

Leu Glu Met Asn Ser Leu Arg Val Glu Asp Thr Ala Val Tyr Tyr Cys
                85                  90                  95

Ala Arg Asp Arg Leu Arg Asn Trp Asn Asp Asp Ala Phe Asp Ile Trp
            100                 105                 110

Gly Pro Gly Thr Met Val Thr Val Ser Ser
        115                 120

<210>  88
<211>  119
<212>  PRT
<213>  Artificial Sequence

<220>
<223>  Antigen binding site or immunoglobulin or immunoglobulin fragment
       specific for Phl p 6

<400>  88

Ser Tyr Glu Leu Thr Gln Pro Ala Ser Val Ser Gly Ser Pro Gly Gln
1               5                   10                  15

Ser Ile Thr Ile Ser Cys Thr Gly Thr Pro Ser Asp Ile Gly Thr Tyr
            20                  25                  30

Pro Tyr Val Ser Trp Tyr Gln Gln His Pro Asp Arg Ala Pro Lys Leu
        35                  40                  45

Leu Ile Tyr Gly Val Thr Asn Arg Pro Ser Gly Val Ser Asn Arg Phe
    50                  55                  60

Ser Gly Ser Lys Ser Gly Asn Thr Ala Ser Leu Thr Ile Ser Glu Leu
65                  70                  75                  80

Gln Ala Glu Asp Glu Ala Asp Tyr Tyr Cys Ser Ser Tyr Thr Leu Thr
                85                  90                  95

Ser Ile Val Val Phe Gly Gly Gly Thr Lys Leu Thr Val Leu Gly Asp
            100                 105                 110

Tyr Lys Asp Asp Asp Asp Lys
        115

<210>  89
<211>  8
<212>  PRT
<213>  Artificial Sequence

<220>
<223>  Antigen binding site or immunoglobulin or immunoglobulin fragment
       specific for Phl p 6

<400>  89

Gly Phe Ile Phe Ser Asp His Tyr
1               5

<210>  90
<211>  8
<212>  PRT
<213>  Artificial Sequence

<220>
<223>  Antigen binding site or immunoglobulin or immunoglobulin fragment
       specific for Phl p 6

<400>  90

Ile Ser Met Thr Ser Ser Tyr Thr
1               5

<210>  91
<211>  15
<212>  PRT
<213>  Artificial Sequence

<220>
<223>  Antigen binding site or immunoglobulin or immunoglobulin fragment
       specific for Phl p 6

<400>  91

Ala Arg Asp Arg Leu Arg Asn Trp Asn Asp Asp Ala Phe Asp Ile
1               5                   10                  15

<210>  92
<211>  9
<212>  PRT
<213>  Artificial Sequence

<220>
<223>  Antigen binding site or immunoglobulin or immunoglobulin fragment
       specific for Phl p 6

<400>  92

Pro Ser Asp Ile Gly Thr Tyr Pro Tyr
1               5


<210>  93
<211>  3
<212>  PRT
<213>  Artificial Sequence

<220>
<223>  Antigen binding site or immunoglobulin or immunoglobulin fragment
       specific for Phl p 6

<400>  93

Gly Val Thr
1


<210>  94
<211>  10
<212>  PRT
<213>  Artificial Sequence

<220>
<223>  Antigen binding site or immunoglobulin or immunoglobulin fragment
       specific for Phl p 6

<400>  94

Ser Ser Tyr Thr Leu Thr Ser Ile Val Val
1               5                   10